# EUROPEAN PATENT APPLICATION

(11) **EP 4 686 761 A1**
(43) Date of publication of application: **04.02.2026**
(21) Application number: 24382852.2
(22) Date of filing: 31.07.2024
(51) Int. Cl.: C12P 19/34, C12Q 1/6844, C12Q 1/6855

(54) **LINEAR DNA PRODUCTS PRODUCED FROM MULTIPLE AMPLIFIED DNA PRODUCTS**

(71) Applicant: 4basebio UK Ltd, Cambridge CB24 5QE (GB)
(72) Inventor: LANCKRIET, Heikki, Over, Cambridge, CB24 5QE (GB); WALKER, Amy, Over, Cambridge, CB24 5QE (GB); BOUCHAREB, Amine, Over, Cambridge, CB24 5QE (GB); MAHADEVA, Tejaswini, Over, Cambridge, CB24 5QE (GB); PAVLICKOVA, Milena, Over, Cambridge, CB24 5QE (GB); PICHER, Ángel, Cantoblanco, Madrid, E-28049 (ES)
(74) Representative: Boult Wade Tennant LLP

(57) **Abstract**

The invention provides methods for producing a linear deoxyribonucleic acid (DNA) product comprising a linear portion of each of at least two amplified DNA products (e.g. a linear portion of a first amplified DNA product and a linear portion of a second amplified DNA product). The invention also provides linear DNA products comprising a linear portion of each of at least two amplified DNA products and uses thereof.

## Description

### TECHNICAL FIELD

The invention relates to methods for producing a linear deoxyribonucleic acid (DNA) product comprising a linear portion of each of at least two amplified DNA products (e.g. a linear portion of a first amplified DNA product and a linear portion of a second amplified DNA product). The method of the invention produces the linear DNA product in a single reaction volume (or single contiguous aqueous volume) comprising the at least two amplified DNA products, an endonuclease and a ligase. The invention also relates to linear DNA products comprising a linear portion of each of at least two amplified DNA products and uses thereof.

### BACKGROUND

There are a wide range of uses for large and/or complex DNA products including both research applications and therapeutic applications such as gene therapies and the generation of DNA and RNA-based vaccines. There are a variety of templates that can be used as a source of DNA for amplification, including plasmids, cosmid vectors, fosmid vectors, bacterial artificial chromosomes (BACs), yeast artificial chromosomes (YACs) (Bajpai B. High Capacity Vectors. Advances in Biotechnology. 2013 Oct 22:1-10. doi: 10.1007/978-81-322-1554-7_1. PMCID: PMC7120981) and linear DNA products (WO2023/006978 A1). Often, bacterial fermentation processes are used for the large-scale amplification of plasmid DNA for production of DNA products for industrial or commercial purposes.

Notwithstanding the availability of a variety of different DNA templates, frequently the sequence composition and/or size of the desired product makes it difficult to completely and/or faithfully amplify it from its source. In particular, the amplification of large regions (e.g. >15kb), GC-rich regions, regions with repeated sequences and homopolymer regions present significant challenges. This means that generating significant quantities of DNA products including such sequence regions is often difficult. There exists a need for methods for completely and/or faithfully amplifying large and/or complex DNA products in a form that is stable and suitable for both research and therapeutic applications.

### DESCRIPTION

The inventors have developed methods for producing a linear deoxyribonucleic acid (DNA) product that is difficult to completely and/or faithfully amplify from a single DNA template molecule.

The invention provides a method for producing a linear deoxyribonucleic acid (DNA) product. The invention is based on producing a linear DNA product comprising a linear portion of a first amplified DNA product and a linear portion of the second amplified DNA product. The method of the invention produces the linear DNA product in a single reaction volume (or single contiguous aqueous volume) comprising a first amplified DNA product, a second amplified DNA product, an endonuclease and a ligase. Thus, the method for producing a linear DNA product comprises: a) forming a single contiguous aqueous volume comprising a first amplified DNA product, a second amplified DNA product, an endonuclease and a ligase; and b) incubating the single contiguous aqueous volume to generate the linear DNA product, wherein the linear DNA product comprises a linear double-stranded region, wherein the linear double-stranded region comprises a linear portion of the first amplified DNA product and a linear portion of the second amplified DNA product. Preferably, the first amplified DNA product and/or the second amplified DNA product is/are product(s) of rolling circle amplification (RCA). Preferably, the first amplified DNA product is a first concatemer and/or the second amplified DNA product is a second concatemer.

The invention provides a method for producing a linear deoxyribonucleic acid (DNA) product, wherein the method comprises:
a) amplifying a first DNA template molecule to generate a first amplified DNA product, wherein the first DNA template molecule comprises at least one cleavable target sequence (e.g. endonuclease target sequence), and amplifying a second DNA template molecule to generate a second amplified DNA product, wherein the second DNA template molecule comprises at least one cleavable target sequence (e.g. endonuclease target sequence);
b) forming a single contiguous aqueous volume comprising the first amplified DNA product, the second amplified DNA product, an endonuclease and a ligase; and
c) incubating the single contiguous aqueous volume to generate the linear DNA product, wherein the linear DNA product comprises a linear double-stranded region, wherein the linear double-stranded region comprises a linear portion of the first amplified DNA product and a linear portion of the second amplified DNA product.

The invention provides a method for producing a linear deoxyribonucleic acid (DNA) product, wherein the method comprises:
a) amplifying a first DNA template molecule to generate a first amplified DNA product, wherein the first DNA template molecule comprises at least one cleavable target sequence (e.g. endonuclease target sequence) and wherein the first amplified DNA product is a first concatemer, and amplifying a second DNA template molecule to generate a second amplified DNA product, wherein the second DNA template molecule comprises at least one cleavable target sequence (e.g. endonuclease target sequence) and wherein the second amplified DNA product is a second concatemer;
b) forming a single contiguous aqueous volume comprising the first amplified DNA product, the second amplified DNA product, an endonuclease and a ligase; and
c) incubating the single contiguous aqueous volume to generate the linear DNA product, wherein the linear DNA product comprises a linear double-stranded region, wherein the linear double-stranded region comprises a linear portion of the first amplified DNA product and a linear portion of the second amplified DNA product.

The invention provides a method for producing a linear deoxyribonucleic acid (DNA) product, wherein the method comprises:
a) amplifying a first DNA template molecule by rolling circle amplification to generate a first amplified DNA product, wherein the first DNA template molecule comprises at least one cleavable target sequence (e.g. endonuclease target sequence) and wherein the first amplified DNA product is a first concatemer, and amplifying a second DNA template molecule by rolling circle amplification to generate a second amplified DNA product, wherein the second DNA template molecule comprises at least one cleavable target sequence (e.g. endonuclease target sequence) and wherein the second amplified DNA product is a second concatemer;
b) forming a single contiguous aqueous volume comprising the first amplified DNA product, the second amplified DNA product, an endonuclease and a ligase; and
c) incubating the single contiguous aqueous volume to generate the linear DNA product, wherein the linear DNA product comprises a linear double-stranded region, wherein the linear double-stranded region comprises a linear portion of the first amplified DNA product and a linear portion of the second amplified DNA product.

In the methods, the step of incubating the single contiguous aqueous volume to generate the linear DNA product may comprise:
i) digesting the first amplified DNA product with the endonuclease by cleaving the cleavable target sequence (e.g. endonuclease target sequence) to generate the linear portion of the first amplified DNA product;
ii) digesting the second amplified DNA product with the endonuclease by cleaving the cleavable target sequence (e.g. endonuclease target sequence) to generate the linear portion of the second amplified DNA product; and
iii) ligating the linear portion of the first amplified DNA product to the linear portion of the second amplified DNA product to generate the linear double-stranded region.

The linear DNA product may be an open linear DNA product, optionally wherein the linear DNA product comprises a first adaptor molecule at a first end and a second adaptor molecule at a second end.

The linear DNA product may be a closed linear DNA product, optionally wherein the linear double-stranded region is closed at a first end by a first adaptor molecule and closed at a second end by a second adaptor molecule. The linear double-stranded region may be closed at a first end by protelomerase (e.g. TeIN) or closed at a second end by protelomerase (e.g. TeIN), optionally wherein a first adaptor molecule comprises a protelomerase target sequence or a portion thereof and/or a second adaptor molecule comprises a protelomerase target sequence or a portion thereof. The linear double-stranded region may be closed at a first end by a first adaptor molecule and closed at a second end by protelomerase (e.g. TeIN) (optionally wherein a second adaptor molecule comprises a protelomerase target sequence or a portion thereof). The linear double-stranded region may be closed at a first end by protelomerase (optionally wherein a first adaptor molecule comprises a protelomerase target sequence or a portion thereof) and closed at a second end by a second adaptor molecule.

The linear double-stranded region may be a partially closed linear DNA, optionally wherein the linear double-stranded region is closed at a first end by a first adaptor molecule or closed at a second end by a second adaptor molecule. The partially closed linear double-stranded region may be closed at a first end by protelomerase (e.g. TeIN), optionally wherein a first adaptor molecule comprises a protelomerase target sequence or a portion thereof. The partially closed linear double-stranded region may be closed at a secondend by protelomerase (e.g. TeIN), optionally wherein a secondadaptor molecule comprises a protelomerase target sequence or a portion thereof.

The invention provides a method for producing a linear DNA product, wherein the method comprises:
a) amplifying a first DNA template molecule (e.g. by rolling circle amplification) to generate a first amplified DNA product, wherein the first DNA template molecule comprises at least one cleavable target sequence (e.g. endonuclease target sequence), and amplifying a second DNA template molecule (e.g. by rolling circle amplification) to generate a second amplified DNA product, wherein the second DNA template molecule comprises at least one cleavable target sequence (e.g. endonuclease target sequence);
b) forming a single contiguous aqueous volume comprising the first amplified DNA product, the second amplified DNA product, an endonuclease, a ligase, and first and second adaptor molecules; and
c) incubating the single contiguous aqueous volume to generate the linear DNA product, wherein the DNA product comprises a linear double-stranded region, wherein the linear double-stranded region comprises a linear portion of the first amplified DNA product and a linear portion of the second amplified DNA product, and wherein the first adaptor molecule is ligated to a first end of the linear double-stranded region and the second adaptor molecule is ligated to a second end of the linear double-stranded region.

The invention provides a method for producing a linear DNA product, wherein the method comprises:
a) amplifying a first DNA template molecule (e.g. by rolling circle amplification) to generate a first amplified DNA product, wherein the first DNA template molecule comprises at least one cleavable target sequence (e.g. endonuclease target sequence) and wherein the first amplified DNA product is a first concatemer, and amplifying a second DNA template molecule (e.g. by rolling circle amplification) to generate a second amplified DNA product, wherein the second DNA template molecule comprises at least one cleavable target sequence (e.g. endonuclease target sequence) and wherein the second amplified DNA product is a second concatemer;
b) forming a single contiguous aqueous volume comprising the first amplified DNA product, the second amplified DNA product, an endonuclease, a ligase, and first and second adaptor molecules; and
c) incubating the single contiguous aqueous volume to generate the linear DNA product, wherein the DNA product comprises a linear double-stranded region, wherein the linear double-stranded region comprises a linear portion of the first amplified DNA product and a linear portion of the second amplified DNA product, and wherein the first adaptor molecule is ligated to a first end of the linear double-stranded region and the second adaptor molecule is ligated to a second end of the linear double-stranded region.

In the methods, the step of incubating the single contiguous aqueous volume to generate the linear DNA product may comprise:
i) digesting the first amplified DNA product with the endonuclease by cleaving the cleavable target sequence (e.g. endonuclease target sequence) to generate the linear portion of the first amplified DNA product;
ii) digesting the second amplified DNA product with the endonuclease by cleaving the cleavable target sequence (e.g. endonuclease target sequence) to generate the linear portion of the second amplified DNA product; and
iii) ligating the first adaptor molecule to a first end of the linear portion of the first amplified DNA product, ligating the second end of the linear portion of the first amplified DNA product to the first end of the linear portion of the second amplified DNA product and ligating the second end of the linear portion of the second amplified DNA product to the second adaptor molecule.

The invention provides a method for producing a closed linear DNA product, wherein the method comprises:
a) amplifying a first DNA template molecule (e.g. by rolling circle amplification) to generate a first amplified DNA product, wherein the first DNA template molecule comprises at least one cleavable target sequence (e.g. endonuclease target sequence), and amplifying a second DNA template molecule (e.g. by rolling circle amplification) to generate a second amplified DNA product, wherein the second DNA template molecule comprises at least one cleavable target sequence (e.g. endonuclease target sequence);
b) forming a single contiguous aqueous volume comprising the first amplified DNA product, the second amplified DNA product, an endonuclease, a ligase, and first and second adaptor molecules; and
c) incubating the single contiguous aqueous volume to generate the closed linear DNA product, wherein the closed linear DNA product comprises a linear double-stranded region, wherein the linear double-stranded region comprises a linear portion of the first amplified DNA product and a linear portion of the second amplified DNA product, and wherein the linear double-stranded region is closed at a first end by the first adaptor molecule and closed at a second end by the second adaptor molecule.

The invention provides a method for producing a closed linear DNA product, wherein the method comprises:
a) amplifying a first DNA template molecule (e.g. by rolling circle amplification) to generate a first amplified DNA product, wherein the first DNA template molecule comprises at least one cleavable target sequence (e.g. endonuclease target sequence) and wherein the first amplified DNA product is a first concatemer, and amplifying a second DNA template molecule (e.g. by rolling circle amplification) to generate a second amplified DNA product, wherein the second DNA template molecule comprises at least one cleavable target sequence (e.g. endonuclease target sequence) and wherein the second amplified DNA product is a second concatemer;
b) forming a single contiguous aqueous volume comprising the first amplified DNA product, the second amplified DNA product, an endonuclease, a ligase, and first and second adaptor molecules; and
c) incubating the single contiguous aqueous volume to generate the closed linear DNA product, wherein the closed linear DNA product comprises a linear double-stranded region, wherein the linear double-stranded region comprises a linear portion of the first amplified DNA product and a linear portion of the second amplified DNA product, and wherein the linear double-stranded region is closed at a first end by the first adaptor molecule and closed at a second end by the second adaptor molecule.

In the methods, the step of incubating the single contiguous aqueous volume to generate the closed linear DNA product may comprise:
i) digesting the first amplified DNA product with the endonuclease by cleaving the cleavable target sequence (e.g. endonuclease target sequence) to generate the linear portion of the first amplified DNA product;
ii) digesting the second amplified DNA product with the endonuclease by cleaving the cleavable target sequence (e.g. endonuclease target sequence) to generate the linear portion of the second amplified DNA product; and
iii) ligating the first adaptor molecule to a first end of the linear portion of the first amplified DNA product, ligating the second end of the linear portion of the first amplified DNA product to the first end of the linear portion of the second amplified DNA product and ligating the second end of the linear portion of the second amplified DNA product to the second adaptor molecule.

The invention provides a method for producing an open linear DNA product, wherein the method comprises:
a) amplifying a first DNA template molecule (e.g. by rolling circle amplification) to generate a first amplified DNA product, wherein the first DNA template molecule comprises at least one cleavable target sequence (e.g. endonuclease target sequence), and amplifying a second DNA template molecule (e.g. by rolling circle amplification) to generate a second amplified DNA product, wherein the second DNA template molecule comprises at least one cleavable target sequence (e.g. endonuclease target sequence);
b) forming a single contiguous aqueous volume comprising the first amplified DNA product, the second amplified DNA product, an endonuclease, a ligase, and first and second adaptor molecules; and
c) incubating the single contiguous aqueous volume to generate the open linear DNA product, wherein the linear DNA product comprises a linear double-stranded region, wherein the linear double-stranded region comprises a linear portion of the first amplified DNA product and a linear portion of the second amplified DNA product, and wherein the first adaptor molecule is ligated to a first end of the linear double-stranded region and the second adaptor molecule is ligated to a second end of the linear double-stranded region, and wherein the first and second adaptor molecules are nucleic acid molecules (e.g. DNA molecules) that each comprise one or more nuclease-resistant nucleotides.

The invention provides a method for producing an open linear DNA product, wherein the method comprises:
a) amplifying a first DNA template molecule (e.g. by rolling circle amplification) to generate a first amplified DNA product, wherein the first DNA template molecule comprises at least one cleavable target sequence (e.g. endonuclease target sequence) and wherein the first amplified DNA product is a first concatemer, and amplifying a second DNA template molecule (e.g. by rolling circle amplification) to generate a second amplified DNA product, wherein the second DNA template molecule comprises at least one cleavable target sequence (e.g. endonuclease target sequence) and wherein the second amplified DNA product is a second concatemer;
b) forming a single contiguous aqueous volume comprising the first amplified DNA product, the second amplified DNA product, an endonuclease, a ligase, and first and second adaptor molecules; and
c) incubating the single contiguous aqueous volume to generate the open linear DNA product, wherein the linear DNA product comprises a linear double-stranded region, wherein the linear double-stranded region comprises a linear portion of the first amplified DNA product and a linear portion of the second amplified DNA product, and wherein the first adaptor molecule is ligated to a first end of the linear double-stranded region and the second adaptor molecule is ligated to a second end of the linear double-stranded region, and wherein the first and second adaptor molecules are nucleic acid molecules (e.g. DNA molecules) that each comprise one or more nuclease-resistant nucleotides.

In the methods, the step of incubating the single contiguous aqueous volume to generate the open linear DNA product may comprise:
i) digesting the first amplified DNA product with the endonuclease by cleaving the cleavable target sequence (e.g. endonuclease target sequence) to generate the linear portion of the first amplified DNA product;
ii) digesting the second amplified DNA product with the endonuclease by cleaving the cleavable target sequence (e.g. endonuclease target sequence) to generate the linear portion of the second amplified DNA product; and
iii) ligating the first adaptor molecule to a first end of the linear portion of the first amplified DNA product, ligating the second end of the linear portion of the first amplified DNA product to the first end of the linear portion of the second amplified DNA product and ligating the second end of the linear portion of the second amplified DNA product to the second adaptor molecule.

The invention provides a method for producing a partially closed linear DNA product, wherein the method comprises:
a) amplifying a first DNA template molecule (e.g. by rolling circle amplification) to generate a first amplified DNA product, wherein the first DNA template molecule comprises at least one cleavable target sequence (e.g. endonuclease target sequence), and amplifying a second DNA template molecule (e.g. by rolling circle amplification) to generate a second amplified DNA product, wherein the second DNA template molecule comprises at least one cleavable target sequence (e.g. endonuclease target sequence);
b) forming a single contiguous aqueous volume comprising the first amplified DNA product, the second amplified DNA product, an endonuclease, a ligase, and first and second adaptor molecules; and
c) incubating the single contiguous aqueous volume to generate the partially closed linear DNA product, wherein the partially closed linear DNA product comprises a linear double-stranded region, wherein the linear double-stranded region comprises a linear portion of the first amplified DNA product and a linear portion of the second amplified DNA product, and wherein the first adaptor molecule is ligated to a first end of the linear double-stranded region and the second adaptor molecule is ligated to a second end of the linear double-stranded region, and wherein the first adaptor molecule is a nucleic acid molecule (e.g. a DNA molecule) that comprises one or more nuclease-resistant nucleotides, and wherein the linear double-stranded region is closed at the second end by the second adaptor molecule (e.g. a DNA molecule).

The invention provides a method for producing a partially closed linear DNA product, wherein the method comprises:
a) amplifying a first DNA template molecule (e.g. by rolling circle amplification) to generate a first amplified DNA product, wherein the first DNA template molecule comprises at least one cleavable target sequence (e.g. endonuclease target sequence) and wherein the first amplified DNA product is a first concatemer, and amplifying a second DNA template molecule (e.g. by rolling circle amplification) to generate a second amplified DNA product, wherein the second DNA template molecule comprises at least one cleavable target sequence (e.g. endonuclease target sequence) and wherein the second amplified DNA product is a second concatemer;
b) forming a single contiguous aqueous volume comprising the first amplified DNA product, the second amplified DNA product, an endonuclease, a ligase, and first and second adaptor molecules; and
c) incubating the single contiguous aqueous volume to generate the partially closed linear DNA product, wherein the partially closed linear DNA product comprises a linear double-stranded region, wherein the linear double-stranded region comprises a linear portion of the first amplified DNA product and a linear portion of the second amplified DNA product, and wherein the first adaptor molecule is ligated to a first end of the linear double-stranded region and the second adaptor molecule is ligated to a second end of the linear double-stranded region, and wherein the first adaptor molecule is a nucleic acid molecule (e.g. a DNA molecule) that comprises one or more nuclease-resistant nucleotides, and wherein the linear double-stranded region is closed at the second end by the second adaptor molecule (e.g. a DNA molecule).

In the methods, the step of incubating the single contiguous aqueous volume to generate the partially closed linear DNA product may comprise:
i) digesting the first amplified DNA product with the endonuclease by cleaving the cleavable target sequence (e.g. endonuclease target sequence) to generate the linear portion of the first amplified DNA product;
ii) digesting the second amplified DNA product with the endonuclease by cleaving the cleavable target sequence (e.g. endonuclease target sequence) to generate the linear portion of the second amplified DNA product; and
iii) ligating the first adaptor molecule to a first end of the linear portion of the first amplified DNA product, ligating the second end of the linear portion of the first amplified DNA product to the first end of the linear portion of the second amplified DNA product and ligating the second end of the linear portion of the second amplified DNA product to the second adaptor molecule.

The linear double-stranded region may comprise a linear portion of the first amplified DNA product ligated to a linear portion of the second amplified DNA product. The linear portion of the first amplified DNA product may be ligated directly to the linear portion of the second amplified DNA product. The linear portion of the first amplified DNA product may be ligated indirectly to the linear portion of the second amplified DNA product. The linear portion of the first amplified DNA product may be ligated to a linker sequence that is ligated to the linear portion of the second amplified DNA product.

The first DNA template molecule may be amplified by rolling circle amplification to generate the first amplified DNA product and/or the second DNA template molecule may amplified by rolling circle amplification to generate the second amplified DNA product.

In the methods, the first amplified DNA product and/or the second amplified DNA product generated in step (a) may not be purified prior to step (b). Preferably, the first amplified DNA product and the second amplified DNA product generated in step (a) are not purified prior to step (b).

In the methods, the DNA template molecules (e.g. the first DNA template molecule and the second DNA template molecule) are amplified either (i) separately, or (ii) together in a single contiguous aqueous volume.

The at least one cleavable target sequence may be a restriction endonuclease target sequence and the endonuclease may be a restriction endonuclease. The at least one cleavable target sequence may be a Type IIS restriction endonuclease target sequence and the endonuclease may be a Type IIS restriction endonuclease.

The endonuclease may be an RNA-guided DNA endonuclease.

Any of the methods provided herein may be a cell-free method.

Whilst the methods are described herein with reference to amplifying first and second DNA template molecules, and first and second amplified DNA products; any of the methods may also be performed with more than two DNA template molecules and more than two amplified DNA products.

In such methods, n is the number of DNA template molecules and the number of amplified DNA products. The invention provides a method for producing a linear deoxyribonucleic acid (DNA) product, wherein the method comprises:
a) amplifying each of n DNA template molecules to generate n amplified DNA products, wherein each of the n DNA template molecules comprises at least one cleavable target sequence (e.g. endonuclease target sequence);
b) forming a single contiguous aqueous volume comprising each of the n amplified DNA products, an endonuclease and a ligase; and
c) incubating the single contiguous aqueous volume to generate the linear DNA product, wherein the linear DNA product comprises a linear double-stranded region, wherein the linear double-stranded region comprises a linear portion of each of the n amplified DNA products.

In the methods, the step of incubating the single contiguous aqueous volume to generate the linear DNA product may comprise:
i) digesting each of the n amplified DNA products with the endonuclease by cleaving the cleavable target sequence (e.g. endonuclease target sequence) to generate a linear portion of the each of the n amplified DNA products; and
ii) ligating the linear portion of each of the n amplified DNA products to the linear portion of another of the n amplified DNA products to generate the linear double-stranded region.

As described herein, a first adaptor molecule and a second adaptor molecule may be included in the single contiguous aqueous volume. The first adaptor molecule may be ligated to the first end of the linear double-stranded region and the second adaptor molecule may be ligated to the second end of the linear double-stranded region.

In the methods, n is at least 3, at least 4, at least 5, at least 6, at least 7, at least 8, at least 9, at least 10, at least 15, at least 20, at least 30, at least 40, at least 50, at least 60, at least 70, at least 80, at least 90 or at least 100.

### 1. DNA cassette

The linear DNA product produced by the methods may comprise a DNA cassette. The DNA cassette may be difficult to completely and/or faithfully amplify from a single DNA template molecule. DNA cassettes may be difficult to completely and/or faithfully amplify from a single DNA template molecule due to their size and/or sequence composition (e.g. high GC content and/or presence of repeat regions such as palindromic repeats).

The DNA cassette may be formed of at least two cassette sequences. The DNA cassette may be formed of a first cassette sequence and a second cassette sequence. The DNA cassette may be formed of at least 3, at least 4, at least 5, at least 6, at least 7, at least 8, at least 9, at least 10, at least 15, at least 20, at least 30, at least 40, at least 50, at least 60, at least 70, at least 80, at least 90 or at least 100 cassette sequences. The DNA cassette may comprise cassette sequences that are each amplified from separate DNA template molecules (each of which comprises a cassette sequence). Each of the amplified DNA products may comprise a cassette sequence. Each of the linear portions of the amplified DNA products may comprise a cassette sequence. The linear double-stranded region (or the linear DNA product) may comprise the DNA cassette (i.e. the complete DNA cassette). Alternatively, one or more cassette sequences (or portions thereof) may be provided by the first adaptor molecule and/or the second adaptor molecule.

Each of the cassette sequences may provide a different sequence of the DNA cassette. Alternatively, at least 2, at least 3, at least 4, at least 5, at least 6, at least 7, at least 8, at least 9, at least 10, at least 15, at least 20 or at least 25 cassette sequences may provide the same sequence of the DNA cassette. The DNA cassette may be formed of at least 2, at least 3, at least 4, at least 5, at least 6, at least 7, at least 8, at least 9, or at least 10, at least 15, at least 20 or at least 25 repeats of the same sequence.

In the methods, the first DNA template molecule may comprise a first cassette sequence; and the second DNA template molecule may comprise a second cassette sequence. The linear double-stranded region may comprise the DNA cassette (i.e. the complete DNA cassette).

In the methods, the first DNA template molecule, the first amplified DNA product and the linear portion of the first amplified DNA product may each comprise a first cassette sequence; and the second DNA template molecule, the second amplified DNA product and the linear portion of the second amplified DNA product may each comprise a second cassette sequence. The linear double-stranded region may comprise the DNA cassette (i.e. the complete DNA cassette).

The skilled person would be aware of a range of options for the composition of the DNA cassette and sequence components that might be included in such a cassette. Various non-limiting options for the cassette are described herein.

The DNA cassette may be at least 1, at least 2, at least 3, at least 4, at least 5, at least 6, at least 7, at least 8, at least 9, at least 10, at least 11, at least 12, at least 13, at least 14, at least 15, at least 20, at least 25, at least 30, at least 35, at least 40, at least 45, at least 50 or at least 55 kilobases (kb) in length. The DNA cassette may be 1-55, 2-50, 3-45, 4-40, 5-35, 6-30, 7-25, 8-20, 9-15, 10-14 or 11-13 kb in length.

Each cassette sequence may be at least 500 bp, at least 1 kb, at least 2 kb, at least 3 kb, at least 4 kb, at least 5 kb, at least 6 kb, at least 7 kb, at least 8 kb, at least 9 kb, at least 10 kb, at least 15 kb, at least 20 kb or at least 25 kb. Each cassette sequence may be 500 bp to 25 kb, 1-20 kb, 2-15 kb, 3-10 kb, 4-9 kb, 5-8 kb or 6-7 kb.

The DNA cassette may be formed of at least 3 cassette sequences. Each cassette sequence may be at least 500 bp, at least 1 kb, at least 2 kb, at least 3 kb, at least 4 kb, at least 5 kb, at least 6 kb, at least 7 kb, at least 8 kb, at least 9 kb, at least 10 kb, at least 15 kb, at least 20 kb or at least 25 kb. Each cassette sequence may be 500 bp to 25 kb, 1-20 kb, 2-15 kb, 3-10 kb, 4-9 kb, 5-8 kb or 6-7 kb.

The DNA cassette may be formed of at least 4 cassette sequences. Each cassette sequence may be at least 500 bp, at least 1 kb, at least 2 kb, at least 3 kb, at least 4 kb, at least 5 kb, at least 6 kb, at least 7 kb, at least 8 kb, at least 9 kb, at least 10 kb, at least 15 kb, at least 20 kb or at least 25 kb. Each cassette sequence may be 500 bp to 25 kb, 1-20 kb, 2-15 kb, 3-10 kb, 4-9 kb, 5-8 kb or 6-7 kb.

The DNA cassette may be formed of at least 5 cassette sequences. Each cassette sequence may be at least 500 bp, at least 1 kb, at least 2 kb, at least 3 kb, at least 4 kb, at least 5 kb, at least 6 kb, at least 7 kb, at least 8 kb, at least 9 kb, at least 10 kb, at least 15 kb, at least 20 kb or at least 25 kb. Each cassette sequence may be 500 bp to 25 kb, 1-20 kb, 2-15 kb, 3-10 kb, 4-9 kb, 5-8 kb or 6-7 kb.

The DNA cassette may be formed of at least 6 cassette sequences. Each cassette sequence may be at least 500 bp, at least 1 kb, at least 2 kb, at least 3 kb, at least 4 kb, at least 5 kb, at least 6 kb, at least 7 kb, at least 8 kb, at least 9 kb, at least 10 kb, at least 15 kb, at least 20 kb or at least 25 kb. Each cassette sequence may be 500 bp to 25 kb, 1-20 kb, 2-15 kb, 3-10 kb, 4-9 kb, 5-8 kb or 6-7 kb.

The DNA cassette may be formed of at least 7 cassette sequences. Each cassette sequence may be at least 500 bp, at least 1 kb, at least 2 kb, at least 3 kb, at least 4 kb, at least 5 kb, at least 6 kb, at least 7 kb, at least 8 kb, at least 9 kb, at least 10 kb, at least 15 kb, at least 20 kb or at least 25 kb. Each cassette sequence may be 500 bp to 25 kb, 1-20 kb, 2-15 kb, 3-10 kb, 4-9 kb, 5-8 kb or 6-7 kb.

The DNA cassette may be formed of at least 8 cassette sequences. Each cassette sequence may be at least 500 bp, at least 1 kb, at least 2 kb, at least 3 kb, at least 4 kb, at least 5 kb, at least 6 kb, at least 7 kb, at least 8 kb, at least 9 kb, at least 10 kb, at least 15 kb, at least 20 kb or at least 25 kb. Each cassette sequence may be 500 bp to 25 kb, 1-20 kb, 2-15 kb, 3-10 kb, 4-9 kb, 5-8 kb or 6-7 kb.

The DNA cassette may be formed of at least 9 cassette sequences. Each cassette sequence may be at least 500 bp, at least 1 kb, at least 2 kb, at least 3 kb, at least 4 kb, at least 5 kb, at least 6 kb, at least 7 kb, at least 8 kb, at least 9 kb, at least 10 kb, at least 15 kb, at least 20 kb or at least 25 kb. Each cassette sequence may be 500 bp to 25 kb, 1-20 kb, 2-15 kb, 3-10 kb, 4-9 kb, 5-8 kb or 6-7 kb.

The DNA cassette may be formed of at least 10 cassette sequences. Each cassette sequence may be at least 500 bp, at least 1 kb, at least 2 kb, at least 3 kb, at least 4 kb, at least 5 kb, at least 6 kb, at least 7 kb, at least 8 kb, at least 9 kb, at least 10 kb, at least 15 kb, at least 20 kb or at least 25 kb. Each cassette sequence may be 500 bp to 25 kb, 1-20 kb, 2-15 kb, 3-10 kb, 4-9 kb, 5-8 kb or 6-7 kb.

The DNA cassette (or a cassette sequence) may comprise a GC content of at least 50%, at least 60%, at least 65%, at least 70%, at least 75%, at least 80%, at least 85%, at least 90%, at least 95%, at least 100%. Preferably, the DNA cassette (or a cassette sequence) may have a GC content of at least 60%. More preferably, the DNA cassette (or a cassette sequence) may have a GC content of at least 65%. GC content may refer to the percentage of bases that are either guanine or cytosine.

The DNA cassette (or a cassette sequence) may comprise a repeated sequence. Repeated sequence may refer to a sequence in which two or more identical or extremely similar nucleotide sequences are repeated. The repeated sequence may be a tandem repeat or interspersed repeat. The repeated sequence may be a direct repeat or inverted repeat (e.g. a palindromic repeat such as an inverted terminal repeat (ITR)). Tandem repeats are repeated sequences which are directly adjacent to each other. Interspersed repeats are repeated sequences with intervening sequences, or repeated sequences that are non-adjacent. Direct repeats occur when a nucleotide sequence is repeated with the same directionality. The DNA cassette may comprise a long terminal repeat (LTR). Inverted repeats occur when a nucleotide sequence is repeated in the inverse direction. When there are no nucleotides separating the inverted repeat, the sequence is called a palindromic repeat. The repeated sequence may be at least 2, at least 5, at least 10, at least 25, at least 50, at least 75, at least 100, at least 250, at least 500, at least 750 or at least 1000 nucleotides long. Preferably, the repeated sequence is at least 100 nucleotides long. The repeated sequence may be 2-1000 nucleotides long, 3-500 nucleotides long, 4-250 nucleotides long, 5-100 nucleotides long, 10-1000 nucleotides long, 15-500 nucleotides long, 20-250 nucleotides long, 25-100 nucleotides long. Preferably, the repeated sequence is 4-250 nucleotides long.

The DNA cassette may comprise inverted terminal repeat sequences upstream and downstream of a coding region. The inverted terminal repeat sequences may be symmetrical (i.e. have the same symmetrical three-dimensional organization with respect to each other) or asymmetrical (i.e. have different three-dimensional organization with respect to each other). The inverted terminal repeat sequences may be from the same or different (viral) serotypes. An inverted terminal repeat sequence may comprise a terminal resolution site and a Rep binding site.

The DNA cassette (or a cassette sequence) may comprise a homopolymeric sequence. The DNA cassette (or a cassette sequence) may comprise a homopolymeric sequence at a 5'-end or a 3'-end or both a 5'-end and a 3'-end. The homopolymeric sequence may be a polyA, a polyC, a polyG, or a polyT sequence. The homopolymeric sequence may be between 3-200 nucleotides in length. The homopolymeric sequence may be used to facilitate purification of the linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides. The homopolymeric sequence may be formed (in the DNA cassette) by at least two cassette sequences.

The DNA cassette (or a cassette sequence) may comprise a coding sequence. The coding sequence may be at least 250, at least 500, at least 750, at least 1000, at least 1500, at least 2000, at least 3000, at least 4000, at least 5000, at least 7500, at least 10000, at least 15000 or at least 20000 base pairs (bp) in length. The coding sequence may be 250 to 20000, 500 to 15000, 750 to 10000, 1000 to 7500, 1500 to 5000, or 2000 to 4000 base pairs (bp) in length

The coding sequence may be formed by at least 2, at least 3, at least 4, at least 5, at least 6, at least at least 7, at least 8, at least 9, at least 10, at least 15, at least 20 or at least 25 cassette sequences.

The DNA cassette (or a cassette sequence) may comprise a promoter (or a portion thereof) and a coding sequence. The promoter (or portion thereof) may be operably linked to the coding sequence. The cassette may comprise a promoter, a coding sequence and a 3' untranslated region e.g. a poly(A) tail. The cassette may comprise a promoter, a coding sequence, a 3' untranslated region e.g. a poly(A) tail, and a translational termination sequence. The cassette may comprise a promoter, a ribosome binding site, a coding sequence and a 3' untranslated region e.g. a poly(A) tail. The cassette may comprise a promoter, a ribosome binding site, a coding sequence, a 3' untranslated region e.g. a poly(A) tail, and a translational termination sequence. The cassette may comprise one or more of: a promoter; a ribosome binding site; a coding sequence; a 3' untranslated region e.g. a poly(A) tail; and a translational termination sequence.

The DNA cassette (or a cassette sequence) may comprise a promoter. The promoter may be an inducible or constitutive promoter. The promoter may be a non-specific promoter. The promoter may be a CMV promoter, a CAG promoter, a Rous sarcoma virus (RSV) promoter, an simian virus 40 (SV40) promoter, a mammalian elongation factor 1α (EF1α) promoter, a MPSV (Moloney Murine Sarcoma Virus) promoter, a Human Ubiquitin C (UBC) promoter and/or a human phosphoglycerate kinase hPGK promoter. The promoter may be a tissue-specific promoter. The promoter may be a thyroxine binding globulin (TBG) promoter, a glial fibrillary acidic protein (GFAP) promoter and/or an actin promoter.

The DNA cassette may further comprise an enhancer. The enhancer may be a CMV enhancer, an SV40 enhancer, a hTERT enhancer, an HIV enhancer, an LTR enhancer and/or a GATA enhancer.

The DNA cassette may comprise an insulator. The insulator may be a cis-regulatory element. The insulator may be at least 300, at least 400, at least 500, at least 1000, at least 1500 or at least 2000 nucleotides in length. The insulator may function either as an enhancer-blocker or a barrier, or both.

The DNA cassette may be an expression cassette. The DNA cassette may be a eukaryotic expression cassette e.g. a mammalian expression cassette or a plant expression cassette. The DNA cassette may be a prokaryotic expression cassette e.g. a bacterial expression cassette. The DNA cassette may be a viral expression cassette.

The DNA cassette may further comprise a reporter gene. The reporter gene may be an eGFP reporter gene, a luciferase reporter gene, or a β-glucuronidase reporter gene.

The DNA cassette may additionally comprise a sequence aiding protein expression, e.g. a cap-independent translation element such as an internal ribosome entry site (IRES).

The DNA cassette may comprise the sequence of a gene. The DNA cassette may comprise the sequence for at least two, three, four, or five genes.

The DNA cassette may comprise a sequence encoding an antigen. The DNA cassette may comprise sequences encoding at least 2, at least 3, at least 4, at least 5, at least 6, at least 7, at least 8, at least 9, at least 10, at least 15, at least 20, at least 30, at least 40, at least 50, at least 60, at least 70, at least 80, at least 90 or at least 100 antigens. The antigen(s) may be (a) self-antigen(s). The antigen(s) may be (a) neoantigen(s).

The DNA cassette may comprise (or encode) a repair template (or editing template). The repair template (or editing template) may be for use in CRISPR-Cas mediated homology directed repair (HDR). The DNA cassette may encode a CRISPR guide RNA.

The DNA cassette may further comprise a LoxP sequence, preferably two LoxP sequences. The two LoxP sequences may be oriented in the same direction. In this case, the DNA sequence between the two LoxP sequences may be excised as a circular loop of DNA. The two LoxP sequences may be oriented in opposite directions. In this case, the DNA sequence between the two LoxP sequences may be inverted. Preferably, the two LoxP sequences are in the same orientation (i.e. the same direction) in the cassette. One LoxP sequence may be at a first end of the cassette and the other LoxP sequence may be at a second end of the cassette. One LoxP sequence may be upstream and the other LoxP sequence may be downstream of the other sequence components of the cassette.

The DNA cassette may be a functional cassette i.e. it may have all sequence elements required for expression of a coding sequence.

Any of the sequence components described above in relation to the DNA cassette may be comprised in a single cassette sequence or split across two or more cassette sequences. For example, a coding sequence may be comprised in a single cassette sequence or split across two or more cassette sequences. A promoter may be comprised in a single cassette sequence or split across two or more cassette sequences.

The first cassette sequence may comprise a promoter and the second cassette sequence may comprise a coding sequence. The first cassette sequence may comprise an enhancer and the second cassette sequence may comprise a promoter and a coding sequence. The first cassette sequence may comprise a first gene coding sequence and a first regulatory sequence, and the second cassette sequence may comprise a second gene coding sequence and a second regulatory sequence. The first cassette sequence may comprise a first homology arm, the second cassette sequence may comprise a coding sequence and the third cassette sequence may comprise a second homology arm. The first cassette sequence may comprise a first ITR region, the second cassette sequence may comprise a coding sequence and the third cassette sequence may comprise a second ITR region. The first cassette sequence may comprise a first ITR region, the second cassette sequence may comprise a first coding sequence, the third cassette sequence may comprise a second coding sequence and the fourth cassette sequence may comprise a second ITR region. The first cassette sequence may comprise a first homology arm, the second cassette sequence may comprise a first coding sequence, the third cassette sequence may comprise a second coding sequence and the fourth cassette sequence may comprise a second homology arm. The first cassette sequence may comprise a first homology arm, the second cassette sequence may comprise a regulatory sequence, the third cassette sequence may comprise a linker block (non-RCA product), the fourth cassette sequence may comprise a coding sequence, and the fifth cassette sequence may comprise a second homology arm.

As described herein, the DNA template molecules (each comprising a cassette sequence) may be amplified separately. The separate amplification reactions may introduce different modifications into the amplified DNA products. For example, one amplification reaction may be performed in the presence of one or more reagents that are not included in the other amplification reaction(s). The one or more reagents may be an enzyme (e.g. methyltransferase), one or more modified nucleotides (e.g. nuclease-resistant nucleotides, methylated nucleotides, labelled nucleotides such as Cy3 or Cy5 labelled nucleotides, fluorescein labelled nucleotides, digoxygenin labelled nucleotides, biotin labelled nucleotides, fluorescent dye labelled nucleotides and/or modified nucleotides used for click chemistry), one or more nucleotide analogues (e.g. fluoro bases, deoxyUridine, zeatin riboside, nictotinamide and/or adenine dinucleotide), one or more fluorescently-conjugated nucleotides, and/or one or more non-hydrolyzable nucleotides.

At least one of the cassette sequences may contain one or more modifications that are not present in any of the other cassette sequences of the DNA cassette. The one or more modifications may be selected from methylation, a label such as Cy3 or Cy5, fluorescein, digoxygenin, biotin, a fluorescent dye, and/or modifications used for click chemistry.

### 2. Amplifying DNA template molecules to generate amplified DNA products

The methods may comprise amplifying DNA template molecules (e.g. first and second DNA template molecules) to generate amplified DNA products (e.g. first and second amplified DNA products), wherein the DNA template molecules comprise at least one cleavable target sequence (e.g. endonuclease target sequence).

### a) DNA template molecule

Each DNA template molecule may comprise a sequence of the DNA cassette (i.e. a cassette sequence). For example, the first DNA template molecule may comprise a first cassette sequence and the second DNA template molecule may comprise a second cassette sequence.

The DNA template molecules may each comprise at least one cleavable target sequence. The cleavable target sequence may be an endonuclease target sequence. Thus, each DNA template molecule may comprise at least one endonuclease target sequence. Preferably, each DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a Bbsl, Bsal, BsmBI, BspQl, BtgZl, Esp3l, Sapl, Aarl, Acc36l, AcIWI, Acul, Ajul, Alol, Alw26l, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1l, Bse3DI, BseGI, BseMl, BseMII, BseNI, BseRl, BseXI, Bsgl, BsIFI, BsmAl, BsmFl, Bsml, Bso31l, BspCNI, BspMI, BspPl, BspQl, BspTNI, BsrDl, Bsrl, Bst6l, BstF5l, BstMAI, BstV1l, BstV2l, Bsul, BtgZl, BtsCl, Btsl-v2, BtsMutl, Bvel, Csel, CspCI, Eam1104l, Earl, Ecil, Eco31l, Eco57l, Esp3l, Faql, Faul, Fokl, Gsul, Hgal, Hphl, HpyAV, Lgul, Lmnl, Lsp1109l, Lwel, Mboll, Mlyl, Mmel, Mnll, Mva1269l, NmeAIII, PaqCl, PciSI, Pctl, Plel, Ppsl, Psrl, Schl, SfaNl, Taqll, TspDTI and/or TspGWI target sequence. The at least one cleavable sequence (e.g. endonuclease target sequence) may be a native cleavable sequence (i.e. a cleavable sequence present in the DNA template molecule(s)). Alternatively, the at least one cleavable sequence (e.g. endonuclease target sequence) may be introduced to the DNA template molecule prior to the amplification of the DNA template molecule.

The DNA template molecule used in the methods may be single-stranded or double-stranded. A single stranded DNA template molecule may be generated by denaturing a double stranded DNA template molecule. Thus, the methods may further comprise denaturing a double stranded DNA template molecule to generate a single stranded DNA template molecule. Denaturation may be achieved by any means known in the art including heat and/or chemical treatment (including organic solvents such as DMSO). Preferably, the DNA template molecule is double-stranded.

The DNA template molecule may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template molecule may be (i) a circular DNA molecule obtained from a recombinase reaction, preferably a Cre recombinase reaction, or (ii) a circular DNA molecule obtained from a ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the template DNA molecule may comprise a double-stranded DNA and a single-stranded hairpin loop.

The DNA template molecule may be linear. If the DNA template molecule is linear, prior to amplification (e.g. rolling circle amplification), the DNA template molecule may be circularized to produce a DNA template molecule suitable for use in the methods described herein.

### b) Amplification

Amplification (i.e. the step(s) of amplifying) may be performed: using a primer specific for the DNA template molecule; using a pair of primers specific for the DNA template molecule; or in the presence of a primase (such as *Thermus thermophilus* (*Tth*) PrimPol (*Tth*PrimPol)). Primers specific for the DNA template molecule include single-stranded nucleic acids that are complementary to a target sequence in the DNA template molecule. A first primer of a set of primers may be complementary to the beginning of a target sequence in the DNA template molecule and a second primer of the set of primers may be complementary to the end of the target sequence in the DNA template molecule. In the case of a double-stranded DNA template, a first primer may be complementary to a first strand and a second primer may be complementary to a second strand.

Amplification (i.e. the step(s) of amplifying) may comprise in vitro or in vivo amplification. Preferably, the step(s) of amplifying is/are steps of in vitro amplification. For example, the step(s) of amplifying may be performed by rolling circle amplification (RCA), Multiple Annealing and Looping Based Amplification Cycles (MALBAC), polymerase chain reaction (PCR), nucleic acid sequence-based amplification (NASBA), loop-mediated isothermal amplification (LAMP), helicase-dependent amplification (HDA), multiple displacement amplification (MDA) or recombinase polymerase amplification (RPA). Preferably, amplification comprises isothermal amplification. More preferably, amplification comprises rolling circle amplification.

Rolling circle amplification may be performed without any primers (i.e. in the presence of a primase), or in the presence of a primer or multiple primers. For example, the primer may be a synthetic primer. The primers may be primers specific for a target sequence and/or random primers. Rolling circle amplification may be performed in the presence of a primase. The primase may be *Thermus thermophilus* (*Tth*) PrimPol (*Tth*PrimPol). Preferably, if the rolling circle amplification is performed without any primers, it is performed in the presence of a primase, such as TthPrimPol. If the rolling circle amplification is performed with a primase, it may also be performed in the presence of one or more primers (such as synthetic primers). Thus, rolling circle amplification may be performed with primers and with a primase. Amplification may be performed with a strand-displacing polymerase e.g. Phi29 DNA polymerase or a mutant thereof which retains functionality (e.g. QualiPhi^{®}, a chimeric form of Phi29 DNA polymerase from 4basebio). Amplification may be performed in the presence of nuclease-resistant nucleotide triphosphates, such as phosphorothioated nucleotide triphosphates. The incorporation of nuclease-resistant nucleotide triphosphates may render the amplified DNA product resistant to exonuclease digestion.

### c) Amplified DNA product(s)

Each amplified DNA product may comprise a sequence of the DNA cassette (i.e. a cassette sequence).

For example, the first amplified DNA product may comprise a first cassette sequence and the second amplified DNA product may comprise a second cassette sequence.

The amplified DNA product(s) is/are preferably produced by rolling circle amplification.

The amplified DNA product(s) may be circular or branched.

The amplified DNA product(s) is/are preferably concatemers (e.g. the first amplified DNA product is a first concatemer and the second amplified DNA product is a second concatemer). The concatemers may be generated by rolling circle amplification. The amplified DNA product(s) may each comprise two or more copies of the same cassette sequence. Each amplified DNA product may be a concatemer comprising multiple copies of same cassette sequence.

The amplified DNA product(s) may each comprise double-stranded regions and single-stranded regions. Preferably, the amplified DNA product(s) is/are double-stranded DNA molecules.

Each amplified DNA product may each comprise at least one cleavable sequence. Each cleavable sequence may be an endonuclease target sequence. Thus, the amplified DNA product may comprise at least one endonuclease target sequence. Preferably, each amplified DNA molecule comprises at least two endonuclease target sequences. The endonuclease target sequences may be the same or different. Preferably, the at least one endonuclease target sequence is a restriction endonuclease target sequence. Different restriction endonuclease target sequences are known to the skilled person. The cleavable target sequence may be a Type IIS restriction endonuclease target sequence. For example, the restriction endonuclease target sequence may be a Bbsl, Bsal, BsmBI, BspQl, BtgZl, Esp3l, Sapl, Aarl, Acc36l, AcIWI, Acul, Ajul, Alol, Alw26l, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1l, Bse3DI, BseGI, BseMI, BseMII, BseNI, BseRl, BseXI, Bsgl, BsIFI, BsmAl, BsmFl, Bsml, Bso31l, BspCNI, BspMI, BspPl, BspQl, BspTNI, BsrDl, Bsrl, Bst6l, BstF5l, BstMAI, BstV1l, BstV2l, Bsul, BtgZl, BtsCl, Btsl-v2, BtsMutl, Bvel, Csel, CspCI, Eam1104l, Earl, Ecil, Eco31l, Eco57l, Esp3l, Faql, Faul, Fokl, Gsul, Hgal, Hphl, HpyAV, Lgul, Lmnl, Lsp1109l, Lwel, Mboll, Mlyl, Mmel, Mnll, Mva1269l, NmeAIII, PaqCl, PciSI, Pctl, Plel, Ppsl, Psrl, Schl, SfaNl, Taqll, TspDTI and/or TspGWI target sequence. The at least one cleavable sequence (e.g. endonuclease target sequence) may be a native cleavable sequence (i.e. a cleavable sequence present in the DNA template molecule(s)). Alternatively, the at least one cleavable sequence (e.g. endonuclease target sequence) may be introduced into the DNA template molecule prior to the amplification of the DNA template molecule.

The amplified DNA product(s) may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve an amplification yield of the amplified DNA product(s). The spacer may improve ligation efficiency of first and second adaptor molecules to the linear double-stranded region. The spacer may improve cell transfection yields.

### 3. Forming and incubating a single contiguous aqueous volume

The methods may comprise: forming a single contiguous aqueous volume comprising at least two amplified DNA products (e.g. the first amplified DNA product and the second amplified DNA product), an endonuclease and a ligase; and incubating the single contiguous aqueous volume to generate the linear DNA product, wherein the linear DNA product comprises a linear double-stranded region, wherein the linear double-stranded region comprises a linear portion of each of the amplified DNA products (e.g. a linear portion of the first amplified DNA product and a linear portion of the second amplified DNA product).

### a) Linear portions of the amplified DNA products and the linear double-stranded region

Each linear portion of an amplified DNA product may comprise a sequence of the DNA cassette (i.e. a cassette sequence). For example, the linear portion of the first amplified DNA product may comprise a first sequence of the DNA cassette and the linear portion of the second amplified DNA product may comprise a second sequence of the DNA cassette.

Each amplified DNA product may be digested by an endonuclease to generate a linear portion of the amplified DNA product (e.g. the first amplified DNA product is digested to generate a linear portion of the first amplified DNA product and the second amplified DNA product is digested to generate a linear portion of the second amplified DNA product). Each linear portion of an amplified DNA product may comprise a single cassette sequence. The term "single cassette sequence" as used herein is intended to encompass a product that does not comprise or consist of a plurality of cassette sequences. That is to say that the digested product (i.e. the linear portion of the amplified DNA product) comprises only a single cassette sequence, which may for example comprise a single coding sequence of a gene of interest. The single cassette sequence may not comprise or consist of a plurality of tandem repeat sequences, and/or concatemeric DNA. The term "single cassette sequence" as used herein is intended to encompass a single copy of a sequence of the DNA cassette, for example, a single copy of the coding sequence or a single copy of a promoter sequence. Thus, the "single cassette sequence" may not encompass a cassette sequence that comprises or consists of multiple copies of the same DNA sequence linked in series.

The linear portions of the amplified DNA products may have low dispersity (i.e. be substantially monodisperse). As used herein, the terms "low dispersity" and "monodisperse" are intended to encompass a collection of copies of digested DNA product of substantially the same size, i.e. the same length of the polynucleotide chain. That is to say, each linear portion of an amplified DNA product may vary in size (i.e. number of bases in the polynucleotide chain) from the other linear portions from the same amplified DNA product by less than 10%, preferably by less than 5%.

The linear portion of each amplified DNA product (e.g. the linear portion of the first amplified DNA product and the linear portion of the second amplified DNA product) may be at least 50, at least 100, at least 250, at least 500, at least 750, at least 1000, at least 2000, at least 3000, at least 4000, at least 5000, at least 6000, at least 7000, at least 8000, at least 9000, at least 10000, at least 11000, at least 12000, at least 13000, at least 14000, at least 15000, or at least 20000 base pairs long. Preferably, the linear portion of each amplified DNA product is at least 100 base pairs long. The linear portion of each amplified DNA product (e.g. the linear portion of the first amplified DNA product and the linear portion of the second amplified DNA product) may be 50 to 20000, 100 to 15000, 250 to 14000, 500 to 13000, 750 to 12000, 1000 to 11000, 2000 to 10000, 3000 to 9000, 4000 to 8000, or 5000 to 7000 base pairs long.

As used herein the term "linear double-stranded region" refers to a double stranded DNA comprising a linear portion of each of at least 2 amplified DNA products (i.e. at least a linear portion of the first amplified DNA product and a linear portion of the second amplified DNA product).

The linear double-stranded region may comprise a DNA cassette (i.e. a complete DNA cassette).

The linear portion of each of the amplified DNA products may be ligated to the linear portion of another of the n amplified DNA products to generate the linear double-stranded region.

The linear portion of each of the amplified DNA products may have an overhang. For example, the linear portion of each of the amplified DNA products may comprise a 5' overhang at a first end and a 3' overhang at a second end. Each overhang may have at least 3 nucleotides (preferably from 4 to 6 nucleotides). The 5' overhang of a linear portion of the first amplified DNA product may be complementary to the 3' overhang of a linear portion of the second amplified DNA product (or vice versa). The 5' overhang of a linear portion of the first amplified DNA product may anneal to the 3' overhang of a linear portion of the second amplified DNA product (or vice versa). The 5' overhang of a linear portion of one amplified DNA product may be complementary to the 3' overhang of a linear portion of another amplified DNA product. The 5' overhang of a linear portion of one amplified DNA product may anneal to the 3' overhang of a linear portion of another amplified DNA product. In this way, the linear double-stranded region may be formed of a linear portion of each of at least 2, at least 3, at least 4, at least 5, at least 6, at least 7, at least 8, at least 9, at least 10, at least 15, at least 20, at least 30, at least 40, at least 50, at least 60, at least 70, at least 80, at least 90 or at least 100 amplified DNA products.

Alternatively, the linear portion of each amplified DNA product may have blunt ends. The blunt ends may be ligated together and, in this way, the linear double-stranded region may be formed of a linear portion of each of at least 2, at least 3, at least 4, at least 5, at least 6, at least 7, at least 8, at least 9, at least 10, at least 15, at least 20, at least 30, at least 40, at least 50, at least 60, at least 70, at least 80, at least 90 or at least 100 amplified DNA products.

The first end and the second end of the linear double-stranded region may be resistant to nuclease digestion. Preferably, the first end and the second end of the linear double-stranded region are resistant to exonucleases that cleave the 3'-end nucleotides (e.g. exonuclease III) and/or exonucleases that cleave the 5'-end nucleotides (e.g. exonuclease VIII).

The linear double-stranded region may comprise an overhang. For example, the linear double-stranded region may comprise a 5' overhang or a 3' overhang. The linear double-stranded region may comprise a blunt end or blunt ends. The linear double-stranded region may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 6 nucleotides). The overhang may be in the sense strand or the antisense strand of the linear double-stranded region.

The linear double-stranded region (or a linear portion of an amplified DNA product) may comprise a plurality of nuclease-resistant nucleotides (e.g. phosphorothioated nucleotides) at internal positions in each strand. For example, the linear double-stranded region (or a linear portion of an amplified DNA product) may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 nuclease-resistant nucleotides (e.g. phosphorothioated nucleotides) at internal positions in each strand. Preferably, the linear double-stranded region (or a linear portion of an amplified DNA product) comprises at least 2 nuclease-resistant nucleotides (e.g. phosphorothioated nucleotides) at internal positions in each strand. The internal positions may not be located between the second and penultimate nucleotide of the linear double-stranded region (or DNA cassette).

The first end of the linear double-stranded region may be complementary to a portion of the first adaptor molecule. The second end of the linear double-stranded region may be complementary to a portion of the second adaptor molecule. The first end and/or the second end of the linear double-stranded region may be generated by endonuclease digestion.

### b) Adaptor molecules

The linear DNA product produced by the methods described herein has enhanced resistance to exonuclease digestion (e.g. exonuclease III digestion). The enhanced resistance to exonuclease digestion may extend the life of the linear DNA product in a cell (i.e. the linear DNA product has enhanced resistance to intracellular exonucleases) and in a cell-free system (i.e. the linear DNA product has enhanced resistance to extracellular exonucleases). The enhanced resistance to exonuclease digestion may be provided by appending (e.g. ligating) a first adaptor molecule to a first end of the linear double-stranded region and a second adaptor to a second end of the linear double stranded region. In this regard, the entire content of WO2023/006978 A1 is incorporated herein by reference.

As described herein, a first adaptor molecule and a second adaptor molecule may be included in the single contiguous aqueous volume. The first adaptor molecule may be ligated to the first end of the linear double-stranded region and the second adaptor molecule may be ligated to the second end of the linear double-stranded region.

The first adaptor molecule may comprise a sequence of the DNA cassette (i.e. a cassette sequence). For example, the first adaptor molecule may comprise a promoter or a portion thereof. The second adaptor molecule may comprise a sequence of the DNA cassette (i.e. a cassette sequence). The second adaptor molecule may comprise a poly(A) tail, or a portion thereof.

The first adaptor molecule and/or the second adaptor molecule may comprise a GC content of at least 50%, at least 60%, at least 65%, at least 70%, at least 75%, at least 80%, at least 85%, at least 90%, at least 95%, at least 100%. Preferably, the first adaptor molecule and/or the second adaptor molecule may have a GC content of at least 60%. More preferably, the first adaptor molecule and/or the second adaptor molecule may have a GC content of at least 65%. GC content may refer to the percentage of bases that are either guanine or cytosine.

The first adaptor molecule or the second adaptor molecule may comprise a sequence of a tandem repeat (with the repeat sequence of the tandem repeat being provided by an adjacent cassette sequence of the DNA cassette).

The first adaptor molecule and/or the second adaptor molecule may comprise an enhancer or a terminator.

The first adaptor molecule and/or the second adaptor molecule may comprise a long terminal repeat (LTR) or a repeat sequence thereof.

In the methods, the first adaptor molecule and the second adaptor molecule may both be linear adaptor molecules comprising nuclease resistant nucleotides, and an open linear DNA product may be produced.

In the methods, the first adaptor molecule and the second adaptor molecule may each comprise a hairpin or a stem-loop, and a closed linear DNA product may be produced.

In the methods, the first adaptor molecule may be a linear adaptor molecule comprising nuclease resistant nucleotides and the second adaptor molecule may comprise a hairpin or a stem-loop (or vice versa), and a partially closed linear DNA product may be produced. The partially closed linear DNA product may be a partially covalently closed linear DNA product.

The adaptor molecules may provide protection from digestion by exonucleases that cleave the 3'-end nucleotides (e.g. exonuclease III) and exonucleases that cleave the 5'-end nucleotides (e.g. exonuclease VIII).

The first and second adaptor molecules may be identical molecules, or they may be different molecules. For example, the first adaptor molecule and/or the second adaptor molecule may comprise a hairpin. The first adaptor molecule and/or the second adaptor molecule may be double-stranded linear nucleic acid molecules comprising one or more nuclease-resistant nucleotides. The first adaptor molecule may comprise a hairpin and the second adaptor molecule may be a double-stranded linear nucleic acid molecule comprising one or more nuclease-resistant nucleotides. The linear DNA product produced by the methods described herein may be resistant to nuclease (e.g. exonuclease) digestion.

The first adaptor molecule may be a nucleic acid adaptor molecule (e.g. DNA). The second adaptor molecule may be a nucleic acid adaptor molecule (e.g. DNA). The first adaptor molecule and/or the second adaptor molecule may be a synthetic adaptor molecule. The first adaptor molecule and/or the second adaptor molecule may not be a plasmid or a vector DNA.

The first adaptor molecule and/or the second adaptor molecule may comprise an overhang. For example, the first adaptor molecule and/or the second adaptor molecule may comprise a 5' overhang or a 3' overhang. The first adaptor molecule and/or the second adaptor molecule may comprise a blunt end. The overhang may have at least 3 nucleotides (preferably from 4 to 6 nucleotides). The overhang of the first adaptor molecule and/or the second adaptor molecule may be complementary to the first and/or second end of the linear double-stranded region. The overhang of the first adaptor molecule and/or the second adaptor molecule may anneal to the first and/or second end of the linear double-stranded region.

The first adaptor molecule and/or the second adaptor molecule may comprise a self-complementary element which creates a loop, such as a hairpin loop or a stem-loop. Thus, the first adaptor molecule may comprise a hairpin or a stem-loop. The second adaptor molecule may comprise a hairpin or a stem-loop. Both the first and second adaptor molecules may comprise a hairpin or a stem-loop. The adaptor molecules may each comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4 to 6 nucleotides. Each end of the linear double-stranded region may comprise a 3' or a 5' overhang.

The first adaptor molecule and/or the second adaptor molecule may comprise a single-stranded portion. The single-stranded portion may form a hairpin or a stem-loop. Thus, the first adaptor molecule and/or the second adaptor molecule may comprise a loop portion. The single-stranded portion may comprise at least 2, at least 3, at least 4, at least 5, at least 6, at least 7, at least 8, at least 9, at least 10, at least 15, at least 20, at least 25, at least 30, at least 35, at least 40, at least 45 or at least 50 nucleotides. The single-stranded portion may comprise 2-50, 3-45, 4-40, 5-35, 6-30, 7-25, 8-20 or 9-15 nucleotides.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first adaptor molecule and/or the second adaptor molecule may comprise a 5' phosphate. The 5' phosphate may facilitate ligation to the linear double-stranded region (which may comprise a 3'-OH group at first and/or second ends). The first adaptor molecule and/or the second adaptor molecule may comprise a 3'-OH. The 3'-OH may facilitate ligation to the linear double-stranded region (which may comprise a 5' phosphate at first and/or second ends).

The first adaptor molecule may comprise a portion (e.g. the overhang) that is complementary to the first end of the linear double-stranded region. The second adaptor molecule may comprise a portion (e.g. the overhang) that is complementary to the second end of the linear double-stranded region. The first adaptor molecule may comprise a portion that anneals to the first end of the linear double-stranded region. The second adaptor molecule may comprise a portion that anneals to the second end of the linear double-stranded region. The first adaptor molecule may comprise a portion that is complementary and anneals to the first end of the linear double-stranded region. The second adaptor molecule may comprise a portion that is complementary and anneals to the second end of the linear double-stranded region.

The portion that is complementary or anneals to the first or second end of the linear double-stranded region may be a 5' overhang or a 3' overhang of the first and/or second adaptor molecule. The overhang of the first adaptor molecule may be complementary to the first end of the linear double-stranded region and/or the overhang of the second adaptor molecule may be complementary to the second end of the linear double-stranded region. The overhang of the first adaptor molecule may anneal to the first end of the linear double-stranded region and/or the overhang of the second adaptor molecule may anneal to the second end of the linear double-stranded region. The overhang of the first adaptor molecule may be complementary to and anneal to the first end of the linear double-stranded region and/or the overhang of the second adaptor molecule may be complementary to and anneal to the second end of the linear double-stranded region.

The first adaptor molecule and/or the second adaptor molecule may not comprise a Type IIS endonuclease target sequence. The first adaptor molecule and/or the second adaptor molecule may not comprise Bbsl, Bsal, BsmBI, BspQl, BtgZl, Esp3l,Sapl, Aarl, Acc36l, AcIWI, Acul, Ajul, Alol, Alw26l, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1l, Bse3DI, BseGI, BseMl, BseMII, BseNI, BseRl, BseXI, Bsgl, BsIFI, BsmAl, BsmFl, Bsml, Bso31l, BspCNI, BspMI, BspPl, BspQl, BspTNI, BsrDl, Bsrl, Bst6l, BstF5l, BstMAI, BstV1l, BstV2l, Bsul, BtgZl, BtsCl, Btsl-v2, BtsMutl, Bvel, Csel, CspCI, Eam1104l, Earl, Ecil, Eco31l, Eco57l, Esp3l, Faql, Faul, Fokl, Gsul, Hgal, Hphl, HpyAV, Lgul, Lmnl, Lsp1109l, Lwel, Mboll, Mlyl, Mmel, Mnll, Mva1269l, NmeAIII, PaqCl, PciSI, Pctl, Plel, Ppsl, Psrl, Schl, SfaNl, Taqll, TspDTI and/or TspGWI target sequences.

The first adaptor molecule and/or the second adaptor molecule may comprise one or more locked nucleic acids (LNAs).

The first adaptor molecule and/or the second adaptor molecule may comprise one or more nuclease-resistant nucleotides (i.e. protected nucleotides), such as phosphorothioated nucleotides. The nuclease-resistant nucleotides may be located in the single-stranded portion (e.g. hairpin portion) or the double-stranded portion of the adaptor molecules. The nuclease-resistant nucleotides may be located in the overhang portion of the adaptor molecules.

The first adaptor molecule and/or the second adaptor molecule may confer resistance to 3'-end exonuclease digestion (e.g. by exonuclease III) and/or 5'-end exonuclease digestion (e.g. by exonuclease VIII).

The closing of the linear double-stranded region at the first end may generate a first closed end of the closed linear DNA product. The closing of the linear double-stranded region at the second end may generate a second closed end of the closed linear DNA product. The first closed end and the second closed end of the closed linear DNA product may be resistant to nuclease digestion. The nuclease digestion may be exonuclease digestion. Preferably, the first closed end and the second closed end of the closed linear DNA product confer resistance to 3'-end exonuclease digestion (e.g. by exonuclease III) and/or 5'-end exonuclease digestion (e.g. by exonuclease VIII).

The first and second adaptor molecules may comprise one or more nuclease-resistant nucleotides (e.g. phosphorothioated nucleotides), such that, once the adaptor molecules are appended (e.g. ligated) to the linear double-stranded region, the linear DNA product is resistant to nuclease digestion or has improved or enhanced resistance to nuclease digestion. The linear DNA product may be resistant to 3'-end exonuclease digestion (e.g. by exonuclease III) and/or 5'-end exonuclease digestion (e.g. by exonuclease VIII).

Each adaptor molecule may comprise a plurality of phosphorothioated nucleotides. For example, each adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

Each adaptor molecule may be double-stranded (e.g. double-stranded DNA). Each adaptor molecule may comprise a portion that is double-stranded (e.g. double-stranded DNA).

The first and/or second adaptor molecules may each comprise at least 3, at least 4, at least 5, at least 6, at least 7, at least 8, at least 9, at least 10, at least 11, at least 12, at least 13, at least 14, at least 15, or at least 16 base pairs.

Each adaptor molecule may comprise a plurality of phosphorothioated nucleotides in each strand. For example, each adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

Each adaptor molecule may comprise a plurality of phosphorothioated nucleotides at internal positions in each strand. For example, each adaptor molecule may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, each adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

Each adaptor molecule may comprise at least 5%, at least 10%, at least 20%, at least 30%, at least 40%, at least 50%, at least 60%, at least 70%, at least 80%, at least 90%, at least 95%, or at least 100% of nuclease-resistant nucleotides.

The nuclease-resistant nucleotides (i.e. nucleotides resistant to exonuclease digestion) may be phosphorothioated nucleotides of at least one type. For example, the at least one type of phosphorothioated nucleotides is α-S-dATP (i.e. 2'-deoxyadenosine-5'-(a-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The adaptor molecules may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The adaptor molecules may comprise at least three types of phosphorothioated nucleotides. For example, the at least three types of phosphorothioated nucleotides are:
(a) α-S-dATP, α-S-dCTP and α-S-dGTP;
(b) α-S-dATP, α-S-dCTP and α-S-dTTP;
(c) α-S-dATP, α-S-dGTP and α-S-dTTP; or
(d) α-S-dCTP, α-S-dGTP and α-S-dTTP.

The adaptor molecules may comprise at least four types of phosphorothioated nucleotides. For example, the at least four types of nuclease-resistant nucleotides are α-S-dATP, α-S-dCTP, α-S-dGTP and α-S-dTTP.

Although the adaptor molecules are described herein as comprising phosphorothioated nucleotides, the skilled person would appreciate that the adaptor molecules may instead comprise any molecules that provide resistance to nuclease digestion (e.g. exonuclease digestion). For example, the adaptor molecules may comprise nuclease-resistant nucleotides i.e. modified nucleotides that provide or increase resistance to nucleases (e.g. exonucleases). The adaptor molecules may comprise a peptide, polypeptide or protein that provides or increases resistance to nucleases (e.g. exonucleases) digestion. The adaptor molecules may comprise 2'-O-methyl nucleotides or 2'-O-methoxyethyl (MOE) nucleotides.

The first adaptor molecule and/or the second adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence.

The functional portion may be a binding molecule. The term "binding molecule" refers to any molecule capable of binding to the linear DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the linear DNA product by binding to capture molecules (e.g. capture antibodies bound by protein-protein interactions). The functional portion may bind to a cell target, for example, a cell receptor.

The functional portion may be a label. The 'label' can be any chemical entity which enables the detection of the double-stranded nucleic acid molecule via physical, chemical and/or biological means. The label may be a chromophore, a fluorophore and/or a radioactive molecule.

The functional portion may be a targeting sequence. The targeting sequence may be a fragment of DNA or RNA of variable length, which is used to target the linear DNA product to a specific location in a cell. The targeting sequence may be used to increase transfection efficiency of non-viral gene delivery by virtue of enhanced nuclear import of the linear DNA product. For example, the targeting sequence may be a DNA nuclear targeting sequence (i.e. a recognition sequence for endogenous DNA-binding proteins), such as a SV40 enhancer sequence (preferably downstream from the DNA cassette).

To facilitate detection and/or quantification of the linear DNA product, the functional portion may comprise a fluorophore, a radioactive compound or a barcode.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeq^{™}, MiSeq^{™} and/or Genome Analyzer^{™} sequencing systems), Oxford Nanopore^{™} Technologies (e.g. the MinION sequencing system), Ion Torrent^{™} (e.g. the Ion PGM^{™} and/or Ion Proton^{™} sequencing systems), Pacific Biosciences (e.g. the PACBIO RS II sequencing system); Life Technologies^{™} (e.g. a SOLiD sequencing system), Roche (e.g. the 454 GS FLX+ and/or GS Junior sequencing systems), or any other sequencing platform of interest.

The first adaptor molecule and/or the second adaptor molecule may comprise an inverted terminal repeat (ITR) sequence. The inverted terminal repeat sequences of the first adaptor molecule and the second adaptor molecule may be symmetrical (i.e. have the same symmetrical three-dimensional organization with respect to each other) or asymmetrical (i.e. have different three-dimensional organization with respect to each other). The inverted terminal repeat sequences of the first adaptor molecule and the second adaptor molecule may be from the same or different (viral) serotypes. An inverted terminal repeat sequence may comprise a terminal resolution site and a Rep binding site.

The first adaptor molecule and/or the second adaptor molecule may comprise an aptamer.

### c) Endonuclease and ligase

The endonuclease may be a restriction endonuclease (such as a Type II restriction endonuclease),

RNA-guided DNA endonuclease, meganuclease or homing endonuclease. The Type II restriction endonuclease may be a Type IIP restriction endonuclease (e.g. EcoRl, Hindlll, BamHI or Notl) or a Type IIS restriction endonuclease (e.g. Bsal, Bbvl, Fokl or Sapl). The RNA-guided DNA endonuclease may be an endonuclease of Class 2 e.g. Class 2 Type V. The RNA-guided DNA endonuclease may be Cas12a. The RNA-guided DNA endonuclease may be Cpf1 or Mad7. Preferably, the RNA-guided DNA endonuclease is Cpf1. The homing endonuclease may be I-Ceul, I-Scel, PI-Pspl or PI-Scel.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQl, BtgZl, Esp3l, Sapl, Aarl, Acc36l, AcIWI, Acul, Ajul, Alol, Alw26l, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1l, Bse3DI, BseGI, BseMl, BseMll, BseNI, BseRl, BseXl, Bsgl, BsIFI, BsmAl, BsmFl, Bsml, Bso31l, BspCNI, BspMI, BspPl, BspQl, BspTNI, BsrDl, Bsrl, Bst6l, BstF5l, BstMAI, BstV1l, BstV2l, Bsul, BtgZl, BtsCl, Btsl-v2, BtsMutl, Bvel, Csel, CspCI, Eam1104l, Earl, Ecil, Eco31l, Eco57l, Esp3l, Faql, Faul, Fokl, Gsul, Hgal, Hphl, HpyAV, Lgul, Lmnl, Lsp1109l, Lwel, Mboll, Mlyl, Mmel, Mnll, Mva1269l, NmeAIII, PaqCl, PciSI, Pctl, Plel, Ppsl, Psrl, Schl, SfaNI, Taqll, TspDTI and/or TspGWI restriction enzyme.

Type IIS restriction endonucleases cleave the amplified DNA product outside of the recognition sequence (i.e. an endonuclease target sequence), which means that the recognition sequence (i.e. endonuclease target sequence) is not included in the linear DNA product.

The endonuclease may be at least 2, at least 3, at least 4 or at least 5 different endonucleases.

The ligase may be a DNA ligase, such as a T4 DNA ligase, T7 DNA ligase, mammalian DNA ligase I, III and IV; Taq DNA ligase, Tth DNA ligase, or *E. coli* DNA ligase.

### d) Incubating the single contiguous aqueous volume

The step of incubating the single contiguous aqueous volume to generate the linear DNA product may comprise: digesting each of the amplified DNA products (e.g. the first amplified DNA product and the second amplified DNA product) with an endonuclease by cleaving the cleavable target sequence (e.g. endonuclease target sequence) to generate a linear portion of each of the amplified DNA products (e.g. a linear portion of the first amplified DNA product and a linear portion of the second amplified DNA product); and ligating the linear portion of each of the amplified DNA products to the linear portion of another of the amplified DNA products to generate the linear double-stranded region (e.g. ligating the linear portion of the first amplified DNA product to the linear portion of the second amplified DNA product to generate the linear double-stranded region).

The step of incubating the single contiguous aqueous volume may be performed under conditions that promote appending (or linking) of the first and second adaptor molecules to the linear double-stranded region to produce the linear DNA product. The appending may be performed by creating a covalent link between the first and/or second adaptor molecule and the first and/or second end of the linear double-stranded region.

The first adaptor molecule may be appended to a first end of the linear double-stranded region and the second adaptor molecule may be appended to a second end of the linear double-stranded region. The appending (or linking) of the of the first adaptor molecule and/or second adaptor molecule may be performed by hybridization and/or ligation of the first adaptor molecule and/or second adaptor molecule to the end(s) of the linear double-stranded region. The first adaptor molecule may be hybridized and/or ligated to the first end of the linear double-stranded region. The second adaptor molecule may be hybridized and/or ligated to the second end of the linear double-stranded region. The appending of the first adaptor molecule and/or the second adaptor molecule may be performed by both hybridization and ligation of the adaptor molecules to the ends of the linear double-stranded region. The first adaptor molecule may be hybridized and ligated to the first end of the linear double-stranded region. The second adaptor molecule may be hybridized and ligated to the second end of the linear double-stranded region. The hybridization is based on complementarity of a portion of the first and/or second adaptor molecules to the first and/or second end of the linear double-stranded region. The appending may be performed via a linker or spacer molecule which facilitates joining of the adaptor molecule to the first and/or second end of the linear double-stranded region.

The linker or spacer may be at least 10, at least 20, at least 30, at least 40, at least 50, at least 60, at least 70, at least 80, at least 90, at least 100, at least 125, at least 150, at least 175, or at least 200 base pairs long.

The step of incubating the single contiguous aqueous volume may be performed under conditions that promote digestion of the amplified DNA products to produce the linear portions of the amplified DNA products. The digestion of the amplified DNA products to produce the linear portions of the amplified DNA products may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of incubating the single contiguous aqueous volume may be performed under conditions that promote ligation of the linear portions of the amplified DNA products (and optionally the first and second adaptor molecules). The ligation may be at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 90%, or at least 95% efficient. For example, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 90%, or at least 95% of the linear portions of the amplified DNA products may be incorporated into linear DNA products. Preferably, the ligation is at least 15% efficient.

Ligation efficiency may be established based on DNA quantification values before and after the digestion/ligation reaction. Thus, ligation efficiency may be established based on the equation: (starting amplified DNA amount) / (final linear DNA amount) x 100%.

Ligation efficiency may also be established based on DNA quantification values before and after the digestion/ligation reaction and the subsequent exonuclease treatment to remove remaining unprotected DNA molecules and adaptor molecules excess.

For example, the amplified DNA products generated by rolling circle amplification is first quantified so that the amount of the amplified DNA products used as the starting material during the digestion/ligation reaction is known. After all the enzymatic reactions, the linear DNA product is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligation of the linear portions of the amplified DNA products (and, optionally, the first and second adaptor molecules) may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C and the second temperature is 14°C-18°C. Using these conditions the endonuclease may be a Type IIS restriction endonuclease (e.g. Bsal) and the ligase may be T4 DNA ligase, T7 DNA ligase, mammalian DNA ligase I, III and IV; Taq DNA ligase, Tth DNA ligase, or *E. coli* DNA ligase.

The step of incubating the single contiguous aqueous volume may be performed isothermally. The step of incubating the single contiguous aqueous volume may comprise incubating at a constant temperature. The constant temperature promotes simultaneous digestion of the amplified DNA products to produce the linear portions of amplified DNA products and ligation of the linear portions of the amplified DNA products and the first and second adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous volume does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

### 4. Optional steps of the methods

The method may further comprise (after the steps of amplification and before the step of forming a single contiguous aqueous volume) a step of heat-deactivation. The step of heat-deactivation may be performed under conditions sufficient to inactivate the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

The inventors of the present application have surprisingly discovered that large concatemeric products of rolling circle amplification reactions can be used to produce linear DNA products. This is surprising as the product of the rolling circle amplification has high viscosity and typically has to undergo purification steps before it can be utilized for downstream applications.

In the method described herein, after the step of amplifying (step (a)) the step of forming a single contiguous aqueous volume (step (b)) may be performed without purifying the amplified DNA products (e.g. the first amplified DNA product and the second amplified DNA product). That is to say that the step of forming a single contiguous aqueous volume (step (b)) may be performed directly after the step of amplifying (step (a)).

Optionally, the first amplification product and/or the second amplification product may be heat-deactivated. The step of forming a single contiguous aqueous volume (step (b)) may be performed directly after the step of heat-deactivation.

In this regard, the inventors of the present application have surprisingly discovered that large concatemeric products of rolling circle amplification reactions can be used to produce linear DNA products without the need for a purification step. This is surprising as the product of the rolling circle amplification has high viscosity and typically has to undergo purification steps before it can be utilized for downstream applications.

The inventors of the present application have discovered a method which requires very few steps to produce the linear DNA product described herein. The methods described herein are very time efficient. This is, in part, due to the fact that a step of purification is not required after the step(s) of amplifying.

The method described herein in which the step of forming a single contiguous aqueous volume (step (b)) is performed without purifying the amplified DNA products (e.g. the first amplified DNA product and the second amplified DNA product) may produce a higher yield of the linear DNA product when compared to a method in which the amplified DNA products are purified before the step of forming a single contiguous aqueous volume (step (b)).

The method may further comprise, after the step of incubating the single contiguous aqueous volume (step (c)), a step of purifying the linear DNA product.

The method may further comprise, after the step of incubating the single contiguous aqueous volume (step (c)), a step of nuclease digestion. The nuclease digestion may be exonuclease digestion, such as exonuclease I and/or exonuclease III digestion. The step of nuclease digestion may take place before or after the step of purifying the linear DNA product. The step of nuclease digestion may allow for removal of any double-stranded DNA molecules and/or adaptor molecules that have not been used to produce linear DNA products. The method may comprise incubating the single contiguous aqueous volume with a nuclease (e.g. an exonuclease).

The method may comprise: (d) incubating the single contiguous aqueous volume with a nuclease (e.g. an exonuclease); and (e) purifying the linear DNA product. The method may comprise: (d) purifying the linear DNA product; and (e) incubating the product of step (d) with a nuclease (e.g. an exonuclease).

The step of incubating with a nuclease may be performed at a temperature of 5-90°C, 10-80°C, 15-70°C, 20-60°C, 25-50°C, 30-45°C or 35-40°C. The step of incubating with a nuclease may be performed for at least 10, at least 20, at least 30, at least 40, at least 50, or at least 60 min. The step of incubating with a nuclease may be performed at two different temperatures. For example, the step of incubating with a nuclease may be performed at 15-40°C for 10-60 minutes followed by a temperature of 60-90°C for 10-30 min. The higher temperature typically inactivates the nuclease (e.g. exonuclease). Thus, the method may further comprise a step of inactivating the nuclease (e.g. exonuclease). The step of incubating with a nuclease may be performed at 37°C for 30 min and 80°C for 20 min. Preferably, the step of inactivating the nuclease (e.g. exonuclease) is performed at a temperature of 70-80°C. The step of inactivating the nuclease (e.g. exonuclease) may be performed for at least 1, at least 5, at least 10, at least 20 or at least 30 minutes. Preferably, the step of inactivating the nuclease (e.g. exonuclease) is performed for at least 5 minutes.

The method may further comprise contacting the single contiguous aqueous volume (or the product of any of the above steps) with Proteinase K.

### 5. Definitions

As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to a DNA sequence.

As used herein the term "nuclease-resistant nucleotide" or "protected nucleotide" is intended to encompass any type of nucleotide that provides or enhances resistance to nuclease digestion (especially exonuclease digestion).

The nuclease-resistant nucleotides (i.e. nucleotides resistant to exonuclease digestion) may be phosphorothioated nucleotides. As used herein, the term "phosphorothioated nucleotide" refers to a nucleotide that has an altered phosphate backbone, wherein the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

The phosphorothioated nucleotides may be α-S-dATP (i.e. 2'-deoxyadenosine-5'-(a-thio)-triphosphate), α-S-dCTP (i.e. 2'-deoxycytidine-5'-(a-thio)-triphosphate), α-S-dGTP (i.e. 2'-deoxyguanosine-5'-(a-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The nuclease-resistant nucleotides (i.e. nucleotides resistant to exonuclease digestion) may be 2'-O-methyl nucleotides or 2'-O-methoxyethyl (MOE) nucleotides. The nuclease-resistant nucleotides may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

### 6. Linear DNA products

The invention provides a linear DNA product (e.g. an open linear DNA product, a closed linear DNA product or partially closed linear DNA product) as described herein.

The invention provides a linear DNA product (e.g. an open linear DNA product, a closed linear DNA product or partially closed linear DNA product) produced or obtainable by the methods for producing a linear DNA product as described herein.

### 7. Methods for transcription and protein expression

The invention provides a method for in vitro transcription of a linear DNA product, wherein the method comprises contacting the linear DNA product produced or obtainable by the methods described herein with a (RNA) polymerase and producing a transcription product encoded by the linear DNA product.

The invention provides a method for *in vitro* transcription of a linear DNA product, the method comprising:
(a) providing a linear DNA product, wherein the linear DNA product is produced by any of the methods described herein;
(b) contacting the linear DNA product with an RNA polymerase; and
(c) producing a transcription product.

The invention provides a method for in vitro transcription of a linear DNA product, wherein the method comprises:
(a) producing a linear DNA product by any of the methods described herein;
(b) contacting the linear DNA product with a (RNA) polymerase; and
(c) producing a transcription product encoded by the linear DNA product.

The invention provides a method for producing a protein, wherein the method comprises introducing the linear DNA product, produced or obtainable by the methods described herein, into a cell (e.g. a prokaryotic cell or a eukaryotic cell) or a cell-free expression system to generate a protein encoded by the linear DNA product.

The invention provides a method for producing a protein, wherein the method comprises:
(a) producing a linear DNA product by any of the methods described herein; and
(b) introducing the linear DNA product into a cell (e.g. a prokaryotic cell or a eukaryotic cell) or a cell-free expression system to generate a protein encoded by the linear DNA product.

The cell-free expression system may originate from (or be derived from) a prokaryotic cell or eukaryotic cell. For example, the cell-free expression system may originate from (or be derived from) rabbit reticulocytes, wheat germ or *Escherichia coli.*

The cell may be a prokaryotic cell or a eukaryotic cell. The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell. The step of introducing the linear DNA product into a cell may be performed in vivo or in vitro.

The linear DNA product may comprise a DNA cassette. The desired protein might be encoded by the DNA cassette. The step of introducing the linear DNA product into a cell may be performed in vivo or in vitro.

The nuclease-resistant nucleotides may be phosphorothioated nucleotides or any other nuclease-resistant nucleotides described herein.

### 8. Methods for cell transfection and cell transfection compositions

The invention provides a method for cell transfection of a linear DNA product produced or obtainable by any of the methods described herein, into a cell.

The invention provides a method for cell transfection of a linear DNA product into a cell, wherein the method comprises:
(a) producing a linear DNA product by any of the methods described herein;
(b) contacting a cell with the linear DNA product; and
(c) transfecting the linear DNA product into the cytosol of the cell.

The invention provides a cell transfection composition comprising a linear DNA product produced by (or obtainable by) the methods described herein.

The cell transfection composition may or may not comprise a carrier e.g. an agent or a formulation. Preferably, the carrier promotes accumulation of the linear DNA product at a target site and/or protects the linear DNA product from undesirable interactions with biological milieu components and/or protects the linear DNA product from metabolism and/or degradation.

The invention further provides a cell transfection method comprising contacting (in vitro) a cell to be transfected with a linear DNA product produced or obtainable by the methods of the invention, and wherein the linear DNA product is transfected into the cytosol of the cell.

The cell or cells to be transfected may be provided in a cell culture medium (e.g. in a Petri dish, culture vessel or well, etc). The linear DNA product may be added directly to the cell culture medium or the cells may be added to a solution, such as saline, a buffered solution or a cell culture medium, comprising the linear DNA product.

The carrier may be a viral carrier or a non-viral carrier. Viral carriers include a lentiviral vector, an adenoviral vector, a retroviral vector, or an adeno associated viral vector for delivery of the linear DNA product. Non-viral carriers (or vectors) include complexing the linear DNA product with a cationic agent such as a cationic cell penetrating peptide (CPP); a DNA-binding cationic component, such as a polylysine chain; a cationic polymer or dendrimer e.g. polyethylenimine (PEl), poly-D,L-lactide-co-glycolide (PLGA), and a block copolymer of PEG and polylysine, and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the linear DNA product. The carrier may be a nanoparticulate formulation used to encapsulate the linear DNA product.

The carrier may be a modification of the linear DNA product with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded nucleic acid molecule (e.g. using cholesterol and/or α-tocopherol).

The cell transfection composition may or may not comprise an agent selected from a photosensitizing agent and/or a radical initiator e.g. a photoinitiator. Preferably, this agent improves the function of the linear DNA product at a target site and/or protects the linear DNA product from metabolism and/or degradation.

The invention further provides a cell obtainable by the methods of the invention. Thus, the cell may comprise a linear DNA product produced or obtainable by the methods of the invention.

The invention further provides a cell transfected with a linear DNA product produced or obtainable by the methods of the invention.

The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The step of contacting the cell with a linear DNA product may be performed in vivo or in vitro. For example, the linear DNA product may be administered to an organism (e.g. a subject) in need thereof. The organism (e.g. the subject) may be an animal, such as mammal (e.g. a human), a fungus, a micro-organism, or a plant.

Any or all of the linear DNA products described herein may be delivered to a cell via delivery particles such as liposomes, nanoparticles, exosomes, macrovesicles, viral or non-viral vectors. Any or all of the linear DNA products described herein may be delivered to a cell using a gene-gun. Any or all of the linear DNA products described herein may be delivered to a cell by electroporation. Any or all of the linear DNA products described herein may be delivered to a cell by hydrodynamic needle. The linear DNA products described herein may be delivered to a cell without a carrier.

The nanoparticle is preferably a self-assembled nanoparticle. The nanoparticle may be a nanoparticle which is produced by a process in which pre-existing components (e.g. a lipid component, a DNA product described herein) form an organized structure as a consequence of specific, local interactions among the components themselves, without external direction.

The linear DNA product and the lipid component may reversibly interact to form a self-assembled nanoparticle. The linear DNA product and the lipid component may reversibly interact in the self-assembled nanoparticle through intermolecular forces. The linear DNA product and the lipid component may reversibly interact in the self-assembled nanoparticle through non-covalent interactions. The linear DNA product and the lipid component may reversibly interact in the self-assembled nanoparticle through hydrogen bonds, van der Waals, hydrophobic, and/or electrostatic interactions. The linear DNA product and the lipid component may not be conjugated or linked by forces other than inter-molecular forces in the self-assembled nanoparticle.

### 9. Pharmaceutical compositions and methods for producing pharmaceutical compositions

The invention provides a pharmaceutical composition comprising a linear DNA product described herein, and a pharmaceutically acceptable carrier or excipient.

The invention provides a pharmaceutical composition comprising a linear DNA product produced or obtainable by the methods described herein, and a pharmaceutically acceptable carrier or excipient.

The invention provides a method for producing a pharmaceutical composition comprising the linear DNA product, wherein the method comprises performing the methods described herein to produce the linear DNA product and formulating the resulting linear DNA product with a pharmaceutically acceptable carrier or excipient.

Thus, the invention provides a method for producing a pharmaceutical composition, wherein the method comprises:
(a) producing a linear DNA product by any of the methods described herein;
(b) formulating the linear DNA product with a pharmaceutically acceptable carrier or excipient.

The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally or transdermally or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

As used herein, injectable and infusion dosage forms include, but are not limited to, a liposomal injectable, which either consists of or forms liposomes (a lipid bilayer vesicle usually composed of phospholipids which is used to encapsulate the composition); an injection, which includes a sterile preparation intended for parenteral use; an emulsion injection, which includes an emulsion consisting of a sterile, pyrogen-free preparation intended to be administered parenterally; or a lipid complex injection.

For example, the linear DNA product can be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochlear capsule.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a manner that liposomes (a lipid bilayer vesicle usually composed of phospholipids which is used to encapsulate the composition, either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

In another mode of administration, the pharmaceutical composition can be administered in situ, via a catheter or pump. A catheter or pump can, for example, direct the composition into the target location.

The parenteral carrier system may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

### 10. Uses and Applications

The invention provides a use of a linear DNA product as described herein in the production of a viral or non-viral delivery system.

The invention provides a use of a linear DNA product in the production of a viral or non-viral delivery system, wherein the linear DNA product is produced or obtainable by performing the method described herein.

The invention provides a viral or non-viral delivery system comprising a linear DNA product as described herein. The invention provides a viral or non-viral delivery system comprising a linear DNA product, wherein the linear DNA product is produced or obtainable by performing the method described herein.

### Viral vectors

Methods of producing viral vectors, such as AAV vectors, are known in the art. The most widely used method involves the co-transfection of HEK293 by three bacterial plasmids. The first plasmid encodes the *Rep* and *Cap* element, the second plasmid is a helper plasmid, while the third plasmid encodes the genetic payload of interest with inverted terminal repeats (ITRs). There are several issues surrounding the use of plasmids for viral preparation (e.g. AAV), namely: 1) the production of the plasmids is time consuming and costly, 2) there is a difficulty in the propagation of ITR sequences in *E.coli*; 3) the incorporation of the plasmid backbone into the viral capsid (e.g. AAV capsid) might be challenging (e.g. issues with antibiotic resistance markers). Methods of producing viral vectors may be performed using other cell lines. For example, a cell line suitable for production of viral vectors may be a Vero cell, or any other stable cell line. Together these represent the main bottleneck in viral vector production (e.g. AAV production). Thus, the methods described herein provide a linear DNA product suitable for use in production of viral vectors. The linear DNA product overcomes the above issues with plasmid vectors.

The invention provides a method of producing a viral vector, the method comprising introducing the linear DNA product produced or obtainable by the methods described herein into a cell under conditions such that the viral vector is produced. The linear DNA product may encode at least one element required for the production of the viral vector. For example, the linear DNA product may encode Rep and/or Cap elements. The linear DNA product may encode the helper plasmid elements. The linear DNA product may encode Rep, Cap and helper plasmid elements. The linear DNA product may encode a transgene. The method may be an in vivo or in vitro method. The cell may be an animal cell, preferably mammal cell, such as human cell (e.g. HEK293T, HEK293, CAP, CAP-T, or CHO). The cell may be a cell cultured in vitro in a tissue culture cell line.

Preferably, the vector is an AAV vector or lentivirus vector.

The invention also provides a method of delivering the viral vector (e.g. the linear DNA product) to a cell, comprising contacting the viral vector produced by the methods described herein with the cell. The cell may be an animal cell, preferably mammal cell, such as human cell.

The invention also provides a cell obtainable by the methods described herein.

### Non-viral vectors

Methods of producing non-viral vectors, are known in the art. Use of non-viral vectors over viral vectors has several advantages, including the opportunity of repeat dosing due to their non-immunogenicity, an unlimited packaging capacity, and low associated toxicity. Most methods for producing non-viral vectors use plasmid DNA. Thus, the linear DNA product produced or obtainable by the methods described herein is suitable for use in production of non-viral vectors. The linear DNA product produced or obtainable by the methods of the invention overcomes the issues of using plasmid vectors in non-viral vector preparation. For example, the linear DNA product, unlike plasmid DNA, does not comprise a bacterial backbone, allowing more transgene copies per mg of DNA. In addition, the linear DNA product does not comprise antibiotic resistant genes and bacterial contaminants. Moreover, the linear DNA product provides a prolonged transgene expression (due to the presence of exonuclease-resistant nucleotides), and a more cost-effective process of non-viral vector production.

The invention provides a method of delivering the non-viral vector to a cell, comprising contacting the non-viral vector comprising the linear DNA product with the cell. The cell may be an animal cell, preferably mammal cell, such as human cell.

The invention also provides a cell obtainable by the methods described herein.

### General therapeutic and diagnostic uses

The linear DNA product produced by the methods described herein is particularly suitable for use in therapy. The invention provides a linear DNA product as described herein for use in therapy. The invention provides a linear DNA product obtainable by the method described herein for use in therapy. The linear DNA product may encode a sequence of a therapeutic protein, a part of a vaccine, or an element of a genetic engineering mechanism, which is used to treat a disease or infection in a subject.

The invention further provides the linear DNA product as described herein for use as a medicament. The invention further provides the linear DNA produced by the methods described herein for use as a medicament. The invention further provides the linear DNA product obtainable by the methods described herein for use as a medicament. The invention also provides the use of a linear DNA product as described herein for the manufacture of a medicament for treating a disease. The invention also provides the use of a linear DNA product produced by the methods described herein for the manufacture of a medicament for treating a disease. The invention also provides the use of a linear DNA product obtainable by the methods described herein for the manufacture of a medicament for treating a disease.

The linear DNA product may encode a sequence of a therapeutic protein, a part of a vaccine, or an element of a genetic engineering mechanism, which is used to treat a disease or infection in a subject.

The invention further provides the linear DNA product produced by the methods described herein for use in treating a disease.

The invention also provides a method of treating a disease in a subject comprising administering to the subject a linear DNA product described herein. The invention also provides a method of treating a disease in a subject comprising administering to the subject the linear DNA product produced by the methods described herein. The invention also provides a method of treating a disease in a subject comprising administering to the subject the linear DNA product obtainable by the methods described herein. Preferably, the amount of the linear DNA product administered to the subject is a therapeutically active amount.

The linear DNA product described herein may be used to treat any disease or disorder. For example, the linear DNA product may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to treat a genetic disorder. More preferably still, the linear DNA product is used to treat a monogenic disorder. For example, the linear DNA product, may be used to treat sickle cell anaemia, cystic fibrosis, Huntington disease, and Duchenne's Muscular Dystrophy, Haemophilia A, a1-antitrypsin deficiency, primary ciliary dyskinesia, or respiratory distress syndrome of prematurity.

A subject treated with the linear DNA product may receive the linear DNA product in the form of any of the pharmaceutical compositions described herein.

A subject treated with the linear DNA product may receive the linear DNA product in combination with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the linear DNA product into the subject's body as described in more detail above (see "Pharmaceutical compositions and methods for producing pharmaceutical compositions"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the linear DNA product that, when administered to a subject for treating a disease, is sufficient to affect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides the use of the linear DNA product as described herein in a method of diagnosing a disease and/or a disorder. The invention also provides the use of the linear DNA product produced or obtainable by the methods described herein in a method of diagnosing a disease and/or a disorder.

The invention provides the use of the linear DNA product in the "in vitro" diagnosis of a disease. The invention provides the use of the linear DNA product obtainable by the methods described herein in the "in vitro" diagnosis of a disease.

The invention also provides the linear DNA product for use in a method of diagnosis "in vivo" of a disease.

The method may be used to diagnose any disease and/or disorder. For example, the diseases and/or disorders may be selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to diagnose a genetic disorder. More preferably still, the linear DNA product is used to diagnose a monogenic disorder. For example, the linear DNA product may be used to diagnose sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The diagnostic and treatment methods described herein may be in vitro methods or in vivo methods.

The method of diagnosis may rely on the detection and/or quantification of the linear DNA product.

To facilitate detection and/or quantification of the linear DNA product, the linear DNA product may be attached or bound to a functional portion. The functional portion may be any functional portion described herein. For example, the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the linear DNA product. For example, the signal may be measured by flow cytometry and/or fluorescence-activated cell sorting of a linear DNA product attached to a fluorescent probe.

The linear DNA product may be detected by being bound to a capture moiety, for example in a lateral flow assay. In this example, the functional portion is a protein, for example, an antibody specific for the capture moiety. The capture of the antibody attached to the linear DNA product may produce a visual signal (e.g. a band of a different colour).

The invention also provides a method that combines diagnosis of a disease with a treatment of the disease.

### Cell therapy

The products produced by the methods of the invention may be substantially less contaminated than plasmid DNA. In addition, the products produced by the methods of the invention are very simple in nature (e.g. no bacterial backbone), which means that they are typically easy to work with. The pure and simple nature of the products described herein makes them particularly suitable for use in cell therapy. For example, a cell comprising the linear DNA product may be injected or otherwise transplanted into a patient to cause a desired effect. The cell (or cells) may be capable of fighting cancer cells, for example, via cell-mediated immunity in the course of immunotherapy. The cell (or cells) may be grafted to regenerate diseased tissues.

The invention provides the linear DNA product described herein for use in cell therapy. The invention provides the linear DNA product produced or obtainable by the methods described herein for use in cell therapy.

Preferably, cell therapy is ex-vivo cell therapy. The cell may be an animal cell, preferably mammal cell, such as human cell.

The invention also provides a cell obtainable by any methods described herein. The cell may be suitable for use in cell therapy.

### Vaccines

The linear DNA products produced by the methods described herein are particularly suitable for use in vaccine production. A vaccine may comprise a linear DNA product described herein. A vaccine may comprise a linear DNA product produced or obtainable by the methods described herein. Alternatively, the linear DNA product may be used to produce a vaccine, preferably an mRNA-based vaccine. For example, vaccines such as the BioNTech and Moderna mRNA vaccines against severe acute respiratory syndrome coronavirus 2 (SARS-CoV-2). The linear DNA product may be used to produce a seasonal, pandemic, prophylactic or therapeutic vaccine.

Thus, the invention provides the use of the linear DNA product described herein in the production of a vaccine. The invention also provides the use of the linear DNA product produced or obtainable by the methods described herein in the production of a vaccine.

The linear DNA product may encode an antigen, which may cause an immune response in a subject. The subject may be human. Preferably, the antigen is encoded on the DNA cassette. The linear DNA product may encode a neoantigen, which may cause an immune response in a subject. Preferably, the neoantigen is encoded on the DNA cassette.

### CAR-T cells

The invention provides the use of a linear DNA product described herein in the production of a CAR-T cell. The invention provides the use of the linear DNA product produced or obtainable by the methods described herein in the production of a CAR-T cell.

The invention provides a method for producing a genetically engineered CAR-T cell comprising: (a) introducing the linear DNA product described herein into a T cell; and (b) expressing a gene of interest encoded by the linear DNA product. Preferably, the gene of interest is a tumour-specific CAR.

The invention provides a method for producing a genetically engineered CAR-T cell comprising: (a) introducing the linear DNA product obtainable by the methods described herein into a T cell; and (b) expressing a gene of interest encoded by the linear DNA product. Preferably, the gene of interest is a tumour-specific CAR.

The method may further comprise, before step (a) (i.e. introducing the linear DNA product into a T cell), a step of removing mononuclear cells from a patient. Preferably, the step of removing is performed using leukapheresis. Preferably the mononuclear cells are T cells. The method may further comprise, after step (b) (i.e. expressing a gene of interest), a step of returning the genetically engineered cells to the patient.

The invention also provides an engineered T cell obtainable by any methods described herein. The engineered T cell may be suitable for use in CAR T-cell therapy.

### CRISPR delivery

The products produced by the methods described herein are particularly suitable for use in delivery of CRISPR machinery to cells, for example in cell therapy or in vivo therapy.

Various different cargos and delivery vehicles are used commonly for delivery of CRISPR machinery, including physical delivery methods (e.g. microinjection; electroporation), viral delivery methods (e.g. adeno-associated virus (AAV); full-sized adenovirus and lentivirus), and non-viral delivery methods (e.g. liposomes; polyplexes; gold particles).

The linear DNA product may comprise a gene sequence encoding any component of the CRISPR machinery. The linear DNA product may encode all components of the CRISPR machinery.

The linear DNA product may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cpf1 or Mad7). The repair template (or editing template) may introduce at least one mutation (e.g. an insertion, deletion, and/or substitution) in the desired DNA region (i.e. target molecule). The repair template (or editing template) may be at least 10, 20, 30, 40, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, or 10000 base pairs long. The linear DNA product encoding the repair template may be delivered to a cell by a nanoparticle, a non-viral vector or a viral vector, or without the aid of any carrier. The linear DNA product encoding the repair template may be delivered to a cell by electroporation. The linear DNA product encoding the repair template may be delivered to a cell by hydrodynamic needle.

The linear DNA product may comprise (or further comprise) a gene sequence encoding a nuclease protein of a CRISPR system (e.g. Cas9, Cpf1 or Mad7), and/or a guide RNA. The linear DNA product may comprise (or further comprise) a gene sequence encoding a nuclease protein of a CRISPR system (e.g. Cas9, Cpf1, or Mad7). The linear DNA product may comprise (or further comprise) a gene sequence encoding a guide RNA. The linear DNA product may comprise (or further comprise) a gene sequence encoding a nuclease protein of a CRISPR system (e.g. Cas9, Cpf1, or Mad7) and a guide RNA. The linear DNA product may comprise (or further comprise) a gene sequence encoding a genomic target to be modified (e.g. a spacer). The linear DNA product may be ligated to a vector. The vector may comprise a sequence of some of the components of the CRISPR system. The vector may comprise a sequence of guide RNA or a sequence of a part of the guide RNA. If the vector comprises a sequence of a part of the guide RNA, the linear DNA product may comprise the missing sequence part of the guide RNA, so that upon ligation, the ligated vector comprises a full sequence of a guide RNA. The nuclease of the CRISPR system and guide RNA may be encoded on a single vector or on two different vectors. The linear DNA product may encode the nuclease of the CRISPR system and guide RNA. One linear DNA product may encode the nuclease of the CRISPR system, and the other linear DNA product may encode guide RNA.

The linear DNA product may comprise (or further comprise) a gene sequence encoding a nuclease protein of a CRISPR system (e.g. Cas9, Cpf1 or Mad7), and/or a gene sequence encoding a DNA polymerase, a reverse transcriptase, or an exonuclease. The linear DNA product may comprise (or further comprise) a gene sequence encoding a DNA polymerase, a reverse transcriptase or an exonuclease. The linear DNA product may comprise (or further comprise) a gene sequence encoding a nuclease protein of a CRISPR system (e.g. Cas9, Cpf1, or Mad7) and a gene sequence encoding a DNA polymerase, a reverse transcriptase or an exonuclease.

The linear DNA product may be for use in CRISPR-Cas mediated repair by recombination, homology-directed repair, or non-homologous end joining.

If the nuclease of the CRISPR system and the guide RNA are encoded by a different linear DNA product, they may be part of a different or the same delivery mechanism. For example, the linear DNA product encoding the nuclease of the CRISPR system may be delivered to a cell by a first nanoparticle, non-viral vector or viral vector, whereas the linear DNA product encoding the guide RNA may be delivered to a cell by a second nanoparticle, a non-viral vector, or viral vector. For example, the linear DNA product encoding the nuclease of the CRISPR system and the linear DNA product encoding the guide RNA may be delivered to a cell by the same nanoparticle, non-viral vector or viral vector.

If the nuclease of the CRISPR system and the guide RNA are encoded by the same linear DNA product (or by a vector that comprises the linear DNA product) they may be part of the same delivery mechanism. For example, the linear DNA product, or the vector, encoding the nuclease of the CRISPR system and the guide RNA may be delivered to a cell by a nanoparticle, a non-viral vector or a viral vector.

The linear DNA product encoding the nuclease of the CRISPR system and the linear DNA product encoding the guide RNA may be delivered to a cell by electroporation. The linear DNA product encoding the nuclease of the CRISPR system and the linear DNA product encoding the guide RNA may be delivered to a cell by hydrodynamic needle.

Thus, the invention provides the linear DNA product described herein for use in CRISPR system delivery to a cell. The invention provides a method of delivering the CRISPR system to a cell, comprising contacting the linear DNA product described herein with a cell. The invention also provides the linear DNA product obtainable by the methods described herein for use in CRISPR system delivery to a cell. The invention provides a method of delivering the CRISPR system to a cell, comprising contacting the linear DNA product obtainable by the methods described herein with a cell.

The cell may be an animal cell, preferably mammal cell, such as human cell.

The invention also provides a cell obtainable by the methods described herein. The cell is particularly suitable for use in cell therapy and/or in vivo therapy.

### mRNA generation

The linear DNA produced or obtainable by the methods described herein may be used as a template for in vitro transcription (IVT).

The linear DNA product produced or obtainable by the methods described herein may be used in transcription, to generate RNA, preferably mRNA, in vitro or in vivo.

The linear DNA product produced or obtainable by the methods described herein may be used in an in vitro transcription (IVT) reaction to generate mRNA which encodes a gene editing nuclease (e.g. Cas9, Cpf1, or Mad7).

A first linear DNA product may encode a nuclease of the CRISPR system (e.g. Cas9, Cpf1, or Mad7) and second linear DNA product may encode a guide RNA. The first and second DNA products may be used as templates for an IVT reaction to generate an mRNA encoding the nuclease of the CRISPR system and a gRNA.

The linear DNA product may comprise (or further comprise) a gene sequence encoding for therapeutic target and a T7 or SP6 promoter. The therapeutic target may comprise or consist of functional gene, vaccine antigen or neoantigen. The therapeutic target may be at least 30, at least 40, at least 50, at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, or at least 10000 base pairs long.

The linear DNA product may comprise (or further comprise) a gene sequence encoding for any component of self-amplifying mRNA system. The linear DNA product may comprise (or further comprise) a gene sequence encoding for all the components of a self-amplifying mRNA system.

The linear DNA product may comprise (or further comprise) a sequence encoding for tRNA or siRNA.

The linear DNA product encoding the nuclease of the CRISPR system and the linear DNA product encoding the guide RNA may be used to treat any disease or disorder. For example, the linear DNA product encoding the nuclease of the CRISPR system and the linear DNA product encoding the guide RNA may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to treat a genetic disorder. More preferably still, the linear DNA product encoding the nuclease of the CRISPR system and the linear DNA product encoding the guide RNA is used to treat a monogenic disorder. For example, the linear DNA product encoding the nuclease of the CRISPR system and the linear DNA product encoding the guide RNA, may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematu rity.

The linear DNA product may comprise (or further comprise) a sequence encoding for self-amplifying mRNA (SAM). The linear DNA product may comprise (or further comprise) a sequence encoding for tRNA. The linear DNA product may comprise (or further comprise) a sequence encoding for circular mRNA (or circRNA).

### 11. Kits

The invention provides a kit comprising components required to carry out the methods described herein.

The kit may comprise:
(a) a strand displacing polymerase;
(b) an endonuclease;
(c) a ligase; and
(d) optionally first and second adaptor molecules.

The strand-displacing polymerase may be Phi29 DNA polymerase or a mutant thereof which retains functionality (e.g. QualiPhi^{®}, a chimeric form of Phi29 DNA polymerase from 4basebio).

The kit may further comprise an exonuclease e.g. exonuclease I, exonuclease III and/or exonuclease VIII.

The endonuclease may be any endonuclease described herein. Preferably, the endonuclease is a Type IIS restriction enzyme.

The ligase may a DNA ligase, such as a T4 DNA ligase, T7 DNA ligase, mammalian DNA ligase I, III and IV; Taq DNA ligase, Tth DNA ligase, or *E*. *coli* DNA ligase.

Each aspect or embodiment as defined herein may be combined with any other aspect(s) or embodiment(s) unless clearly indicated to the contrary. In particular, any feature indicated as being preferred or advantageous may be combined with any other feature or features indicated as being preferred or advantageous.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

Various publications are cited herein, the disclosures of which are incorporated by reference in their entireties.

### BRIEF DESCRIPTION OF THE DRAWINGS

**FIG. 1** Illustrates the workflow used to obtain the compound DL product (two individual DNA sequences ligated together to form a single construct with adaptors ligated on either end of the construct using a single pot reaction).
**FIG. 2A** **and** **FIG. 2B** Agarose gel (0.8%) image showing the compound DL product from single pot compound DL (digestion and ligation) reaction and exonucleases treatments.
**FIG. 3A** **and** **FIG. 3B** Agarose gel (0.8%) image showing the compound DL product obtained after the compound DL reaction, Proteinase K and exonucleases treatments.

### EXAMPLES

### Example 1: Workflow to obtain the compound DL product

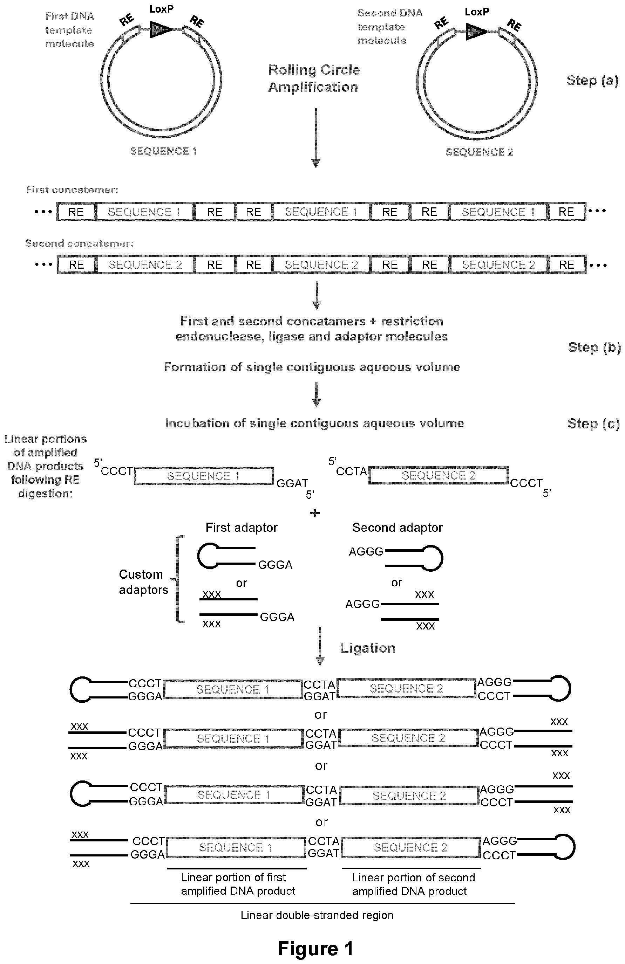

Rolling circle amplification (RCA) was carried out separately on both the first DNA template molecule (sequence 1) and the second DNA template molecule (sequence 2) (as shown in Step (a) of FIG. 1) to generate first and second amplified DNA products, which are concatemers (long DNA molecules that contain the same copy of a DNA sequence linked in series with restriction endonuclease target sequences (denoted "RE") flanking each copy of the DNA sequence. The template DNA molecules were circularized prior to the RCA reaction so that they are compatible with the RCA reaction. The first and second amplified DNA products were combined in single pot compound DL (Digestion and Ligation) reaction along with a restriction endonuclease, ligase, and first and second adaptor molecules (hairpin, or linear adaptor molecules comprising nuclease resistant nucleotides (denoted "xxx" in Figure 1) in a reaction buffer (Step (b) of FIG. 1). A single pot reaction involves simultaneous digestion and ligation reactions whereby complementary overhangs are ligated after digestion by restriction enzymes or endonucleases which are specific to restriction or endonuclease sites flanking the sequences of interest. Concatemers produced via the RCA reactions (first and second concatamers) were digested by a restriction enzyme present in the compound DL reaction to produce first and second linear portions of the amplified DNA products with overhangs (as shown in Step c of FIG. 1). The downstream overhang of the linear portion of the first amplified DNA product is complementary only to the upstream overhang of the linear portion of the second amplified DNA product; in addition, first and second adaptor molecule overhangs are complementary only to the upstream and downstream of the linear portions of the first and second amplified DNA products, respectively. Complementary overhangs are ligated by ligases to produce the compound DL product (as shown in Step c of FIG. 1). The product obtained at the end of the compound DL reaction is referred to as the compound DL product and consists of a linear portion of the first amplified DNA product and a linear portion of the second amplified DNA product ligated together to form a single construct (the linear double-stranded region) with first and second adaptor molecules ligated on either end of the construct (as shown in Step c of FIG. 1). The products of the compound DL reaction may also contain off target ligation products or non-ligated products; therefore, the single pot reaction was subjected to exonuclease treatment to remove any off-target ligation products or non-ligated products that were present.

In addition, to potentially obtain just a band corresponding to the compound DL product with no trace of high molecular weight product in the well after agarose gel analysis, Proteinase K treatment was introduced in between the single pot digestion and ligation step and the exonuclease treatment step to see whether Proteinase K treatment enhances the performance of the overall process by reducing the enzyme burden that is carried on to the exonuclease treatment step.

### Example 2: Production of the compound DL product

Sequence 1 and sequence 2 were amplified in individual RCA reactions. Before the amplification, the DNA sequences were circularized, and then the circularized DNA sequences were first denatured by adding 1 volume of buffer D (400 mM KOH, 10 mM EDTA) and incubating for 3 min at room temperature. The samples were then neutralized by adding 1 volume of buffer N (400 mM HCI, 600 mM Tris-HCI pH 7.5). The RCA conditions were as follows: 1 ml reaction volume, 0.01 ml TruePrime Whole Genome Amplification (WGA) reaction buffer 10x (4basebio), 0.150 mL denatured DNA sample, 0.046 ml TthPrimPol (740 ng/uL), 0.016 mL QualiPhi Phi29DNApol (1000 ng/uL), 0.0025mL PPase (80 ng/uL) and 0.04 mL dNTPs (25 mM). Incubation time and temperature: 20 hours at 30°C, followed by incubation for 10 min at 65°C.

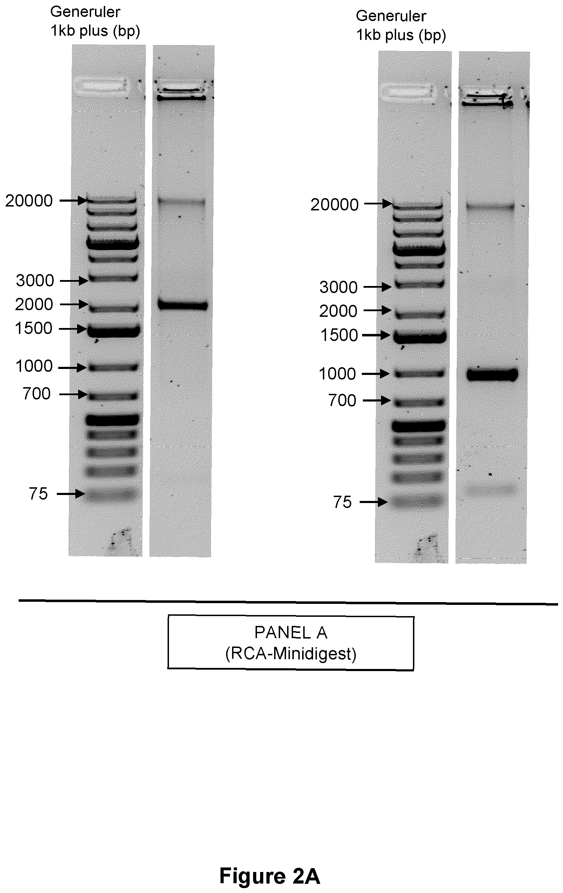

The RCA material aliquot was subjected to Bsal digestion before feeding the material into the compound DL reaction to confirm that the size of the amplified products for sequence 1 and sequence 2 were as expected. FIG. 2A, Panel A shows that upon Bsal digestion, sequence 1 produced a band of ~1000 bp and sequence 2 produced a band of -2000 bp, which was as expected. Following this confirmation the amplified sequences were further processed to produce the compound DL product.

In the compound DL reaction, equivolume of sequence 1 and sequence 2 with an initial concentration of 499.5ng/ul (obtained after amplifying the sequences individually in RCA reactions) were combined in a single pot compound DL reaction to give a final DNA concentration of 240ng/uL in a total volume of 0.2 ml. The components of the compound DL reaction consisted of RCA DNA material (240ng DNA/uL), enzymes (Bsal (3.2 ng/uL 4Basebio Bsal)), ligases (T3 Ligase NEB (4.95 U/uL) or T4 Ligase 4Basebio (8.3 ng/uL) or T7 Ligase NEB (4.95 U/uL)), hairpin adaptors (10x molar excess of adaptor per DNA end), digestion ligation reaction buffer (50 mM Tris HCl [pH 7.5], 10 mM MgCl2, 10 mM DTT) and 10mM ATP. The reaction was incubated for 18 h at 25°C or 37°C.

Sequence 1 is 927 bp, sequence 2 is 1968 bp. When ligated a 2895 bp construct was formed. The final size of the construct was used for calculations of 10x molar excess of the hairpin adaptor required per DNA end. The addition of hairpin adaptors protects the construct from exonuclease activity and any molecules not flanked by hairpin adaptors are digested by the exonucleases.

The digested material and hairpin adaptors were ligated using one of three ligase types: T3 ligase (with a final concentration of 4.95 U/uL), T4 ligase (with a final concentration of 8.3ng/uL) or T7 ligase (with a final concentration of 4.95 U/uL).

Aliquots were taken after the compound DL reaction and the remaining single pot compound DL reaction was further treated with exonuclease I (final concentration of 4.462 ng/uL) and exonuclease III (final concentration of 8.428 ng/uL) and incubated for 2 h at 37°C to digest non-ligated or off target product. After exonuclease treatment, further aliquots were taken for analysis.

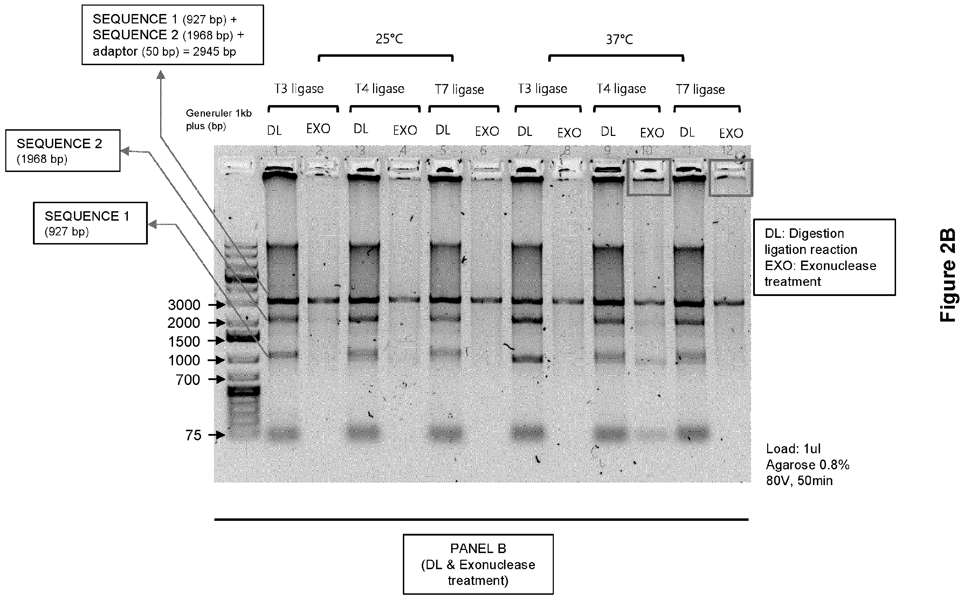

Fluorometer (Qubit) measurements were taken for each of the aliquots taken after the single pot compound DL reaction and after exonuclease treatment. All of the ligase conditions produced a compound DL product of -3000bp. Since the construct (sequence 1 + sequence 2) was flanked by hairpin adaptors, the construct withstood exonuclease treatment. Furthermore, with T7 ligase there was no off-target ligation product carry over after exonuclease treatment at both the 25°C and 37°C incubation temperatures (see FIG. 2B, Panel B, lane 6 and lane 12), whereas the T4 ligase-containing reaction showed carry over of the off-target ligation product of ~1000 bp (sequence 1) at the 25°C incubation temperature (see FIG. 2B, Panel B, lane 4) and the off target ligation products of ~ 1000bp (sequence 1) and ~2000bp at the 37°C incubation temperature (sequence 2) (see FIG. 2B, Panel B, lane 10) after exonuclease treatment.

On the other hand, carry over of the off target ligation product of -2000 bp (sequence 2) was observed for the T3 ligase condition at the 25°C incubation temperature (see FIG. 2B, Panel B, lane 2) but no off-target ligation product was observed at 37°C (see FIG. 2B, Panel B, lane 8) after exonuclease treatment.

**Table 2: Shows the DNA concentration measurement using Qubit and agarose gel densitometry analysis for compound DL, exonuclease treated product.**

| **Incubation temperature °C** | **Ligase type** | **After compound DL (ng/ul)** | **After exonuclease treatment (ng/ul)** | **Qubit ligation efficiency %** | **Agarose gel Densitometry ligation efficiency %** |
|---|---|---|---|---|---|
| 25°C | T3 ligase | 279 | 61.3 | 21.97 | 76.13 |
| | T4 ligase | 284 | 75.15 | 26.46 | 55.85 |
| | T7 ligase | 266 | 73.25 | 27.54 | 65.66 |
| 37°C | T3 ligase | 289 | 55.65 | 19.26 | 73.99 |
| | T4 ligase | 269 | 58.4 | 21.71 | 41.34 |
| | T7 ligase | 257 | 88.5 | 34.44 | 67.24 |

Even though T3 ligase has a higher ligation efficiency of 76.13 % compared to T7 ligase with a ligation efficiency of 65.66%, T7 ligase at 25°C worked the best compared to T3 or T4 ligase as the T7 ligase condition generated reliable data and obtained just the product of interest with no off-target ligation products. Although T3 ligase condition obtained just the product of interest, its Qubit value of 55.65 ng/ul was lower than the T7 ligase Qubit value of 88.5 ng/ul. Again, the agarose gel densitometry ligation efficiency for T7 ligase of 34.44% was higher than the T3 ligase efficiency of 19.26 %. Both the 25°C and 37°C incubation temperatures for the ligase condition data showed that T7 ligase works the best in terms of product yield and ligation efficiency despite changing the incubation temperatures by producing just the product of interest with no off-target ligation products.

### Example 3: Proteinase K treatment between compound DL reaction and exonuclease treatment

Proteinase K treatment was performed between the compound DL reaction and exonuclease treatment. This was based on the assumption that Proteinase K would reduce the enzyme burden carried from the compound DL reaction into the enzymatic purification step and result in a better performance of exonuclease enzymes, producing a purer compound DL product.

Individual rolling circle amplification reactions were performed for each circularised template containing sequence 1 or sequence 2. Before amplification, circularized DNA sequences were first denatured by adding 1 volume of buffer D (400 mM KOH, 10 mM EDTA) and incubating for 3 min at room temperature. Samples were then neutralized by adding 1 volume of buffer N (400 mM HCl, 600 mM Tris-HCl pH 7.5). The conditions for RCA were as follows: 1 ml reaction volume, 0.01 ml TruePrime WGA reaction buffer 10x (4basebio), 0.150 mL denatured DNA sample, 0.046 ml TthPrimPol (740 ng/uL), 0.016 mL QualiPhi Phi29DNApol (1000 ng/uL), 0.0025mL PPase (80 ng/uL) and 0.04 mL dNTPs (25 mM). Incubation time and temperature: 20 hours at 30°C and followed by incubation for 10 min at 65°C.

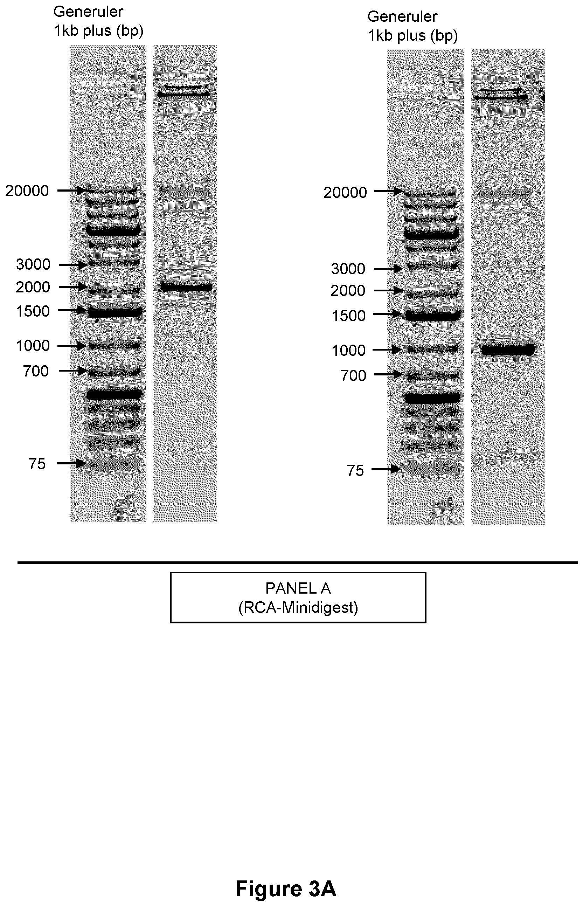

The aliquots of each RCA reaction material were subjected to Bsal digestion and evaluated for expected size in agarose gel analysis before feeding the material into a compound DL reaction. FIG. 3A, Panel A shows that upon Bsal digestion, a band of bp (corresponding to sequence 1) and a band of and -2000 bp (corresponding to sequence 2) were produced, as expected. Upon confirmation these amplified sequences were further processed to produce the compound DL product.

In the compound DL reaction, equivolume of sequence 1 and sequence 2 with an initial concentration of 499.5ng/ul (obtained after amplifying them individually in RCA processes) were combined in a single pot compound DL reaction to give a final DNA concentration of 240ng/uL in total volume of 0.2 ml. The components of the compound DL reaction consisted of: RCA DNA material (240ng DNA/uL), enzymes (either Bsal or another endonuclease with a restriction site present in the RCA concatemer (3.2 ng/uL 4Basebio Bsal)), ligases (T4 Ligase 4Basebio (8.3 ng/uL) or T7 Ligase NEB (4.95 U/uL)), hairpin adaptors (usually 10x molar excess of adaptor per DNA end), digestion ligation reaction buffer (50 mM Tris HCl [pH 7.5], 10 mM MgCl2, 10 mM DTT) and 10mM ATP. The reaction was incubated for 18 h at 37°C.

Sequence 1 is 927 bp, sequence 2 is 1968 bp. When ligated, a 2895 bp construct was formed. This final size of the construct was used for calculations of 10x molar excess of the hairpin adaptor required per DNA end. The addition of hairpin adaptors protects the construct from exonuclease activity and any molecules not flanked by hairpin adaptors are digested by the exonucleases.

Digested material and hairpin adaptors were ligated using one of two ligase types: T4 ligase (with a final concentration of 8.3ng/uL) or T7 ligase (with a final concentration of 4.95 U/uL).

Aliquots were taken after the compound DL reaction and the remaining single pot compound DL reaction mixture was further treated with Proteinase K enzyme (0.4 ug/mL) at 55 °C for 1 h. After the incubation, Proteinase K inhibitor (0.25mM) was added to the reaction to inhibit the activity of Proteinase K and was incubated at room temperature for at least 15 min before the addition of exonuclease to the reaction.

Aliquots were taken after the Proteinase K treatment and rest of the reaction was further treated with exonuclease I (final concentration of 4.5 ng/uL) and exonuclease III (final concentration of 8.4 ng/uL) and incubated for 2 h at 37°C to digest non-ligated or off target product. After the exonuclease treatment, aliquots were taken for analysis.

Qubit measurements were taken for each aliquot after the single pot compound DL reaction and after exonuclease treatment.

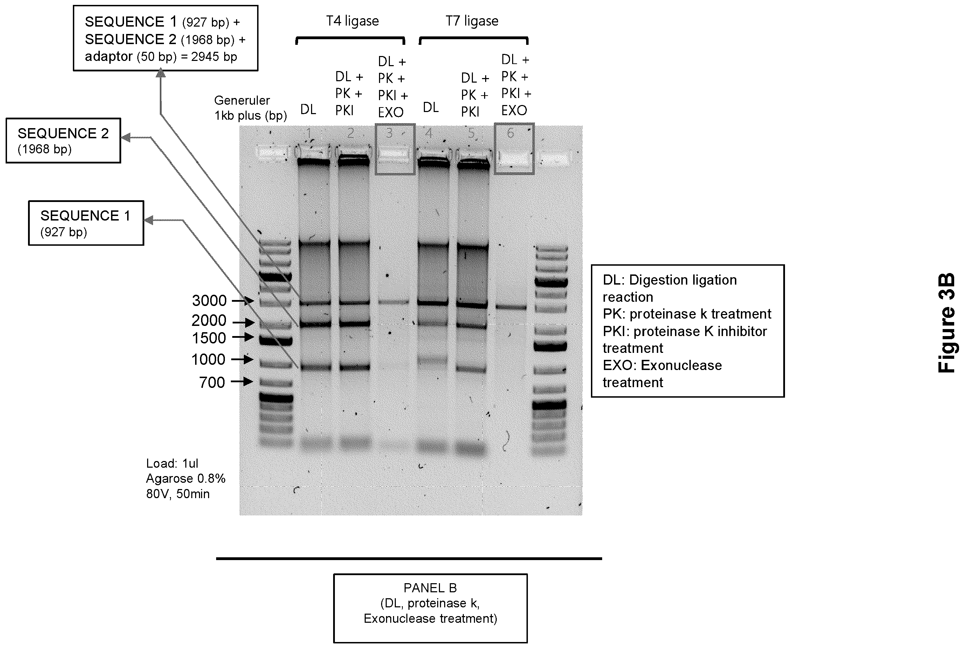

The outcome of the study showed that including Proteinase K treatment could improve the product quality. Both ligase conditions produced a compound DL product of -3000bp. Since the construct (sequence 1 + sequence 2) was flanked by hairpin adaptors, the construct withstood exonuclease treatment. Furthermore, T7 ligase had no off-target ligation product carry over after exonuclease treatment (see FIG. 3B, Panel B, lane 6), whereas the T4 ligase-containing reaction showed carry over of the off-target ligation product of bp (corresponding to sequence 1) (see FIG. 3B, Panel B, lane 3) after exonuclease treatment.

The addition of the Proteinase K step between the compound DL and enzymatic purification steps (see FIG. 3B, Panel B, lane 3 and lane 6) as compared to the protocol without the Proteinase K step (see FIG. 2B, Panel B, boxes in lane 10 and lane 12) reduced the presence of high molecular weight amplified DNA and hence improved linear product purity. This result shows that Proteinase K treatment between the compound DL reaction and exonuclease treatment could be beneficial for producing a better quality compound DL product.

**Table 4: Shows the DNA concentration measurement using Qubit and agarose gel densitometry analysis for compound DL samples treated with Proteinase K before exonuclease treatment.**

| **Incubation temperature** | **Ligases** | **After compound DL (ng/ul)** | **After exonuclease treatment (ng/ul)** | **Qubit ligation efficiency %** | **Agarose gel Densitometry ligation efficiency %** |
|---|---|---|---|---|---|
| 37°C | T4 ligase | 268 | 27.4 | 10.46 | 65.25 |
| | T7 ligase | 260 | 30.7 | 11.3 | 72.42 |

Even though a major difference between T4 and T7 ligase Qubit values and ligation efficiency was not observed, the T7 Ligase condition worked better than theT4 ligase condition as the T7 ligase condition generated reliable data with just the product of interest and no off-target ligation products with a qubit value of 30.7 ng/ul and ligation efficiency of 72.42%

## Claims

1. A method for producing a linear deoxyribonucleic acid (DNA) product, wherein the method comprises:
a) amplifying a first DNA template molecule to generate a first amplified DNA product, wherein the first DNA template molecule comprises at least one cleavable target sequence and wherein the first amplified DNA product is a first concatemer, and amplifying a second DNA template molecule to generate a second amplified DNA product, wherein the second DNA template molecule comprises at least one cleavable target sequence and wherein the second amplified DNA product is a second concatemer;
b) forming a single contiguous aqueous volume comprising the first amplified DNA product, the second amplified DNA product, an endonuclease and a ligase; and
c) incubating the single contiguous aqueous volume to generate the linear DNA product, wherein the linear DNA product comprises a linear double-stranded region, wherein the linear double-stranded region comprises a linear portion of the first amplified DNA product and a linear portion of the second amplified DNA product.

2. The method of claim 1, wherein the linear DNA product is an open linear DNA product, optionally wherein the linear DNA product comprises a first adaptor at a first end and a second adaptor at a second end.

3. The method of claim 1, wherein the linear DNA product is a closed linear DNA product, optionally wherein the linear double-stranded region is closed at a first end by a first adaptor molecule and closed at a second end by a second adaptor molecule.

4. The method of claim 1, wherein the linear double-stranded region is a partially closed linear DNA, optionally wherein the linear double-stranded region is closed at a first end by a first adaptor molecule.

5. The method of claim 1, wherein the method comprises:
a) amplifying a first DNA template molecule to generate a first amplified DNA product, wherein the first DNA template molecule comprises at least one cleavable target sequence and wherein the first amplified DNA product is a first concatemer, and amplifying a second DNA template molecule to generate a second amplified DNA product, wherein the second DNA template molecule comprises at least one cleavable target sequence and wherein the second amplified DNA product is a second concatemer;
b) forming a single contiguous aqueous volume comprising the first amplified DNA product, the second amplified DNA product, an endonuclease, a ligase, and first and second adaptor molecules; and
c) incubating the single contiguous aqueous volume to generate the linear DNA product, wherein the DNA product comprises a linear double-stranded region, wherein the linear double-stranded region comprises a linear portion of the first amplified DNA product and a linear portion of the second amplified DNA product, and wherein the first adaptor molecule is ligated to a first end of the linear double-stranded region and the second adaptor molecule is ligated to a second end of the linear double-stranded region.

6. The method of claim 1, wherein the linear DNA product is a closed linear DNA product, and wherein the method comprises:
a) amplifying a first DNA template molecule to generate a first amplified DNA product, wherein the first DNA template molecule comprises at least one cleavable target sequence and wherein the first amplified DNA product is a first concatemer, and amplifying a second DNA template molecule to generate a second amplified DNA product, wherein the second DNA template molecule comprises at least one cleavable target sequence and wherein the second amplified DNA product is a second concatemer;
b) forming a single contiguous aqueous volume comprising the first amplified DNA product, the second amplified DNA product, an endonuclease, a ligase, and first and second adaptor molecules; and
c) incubating the single contiguous aqueous volume to generate the closed linear DNA product, wherein the closed linear DNA product comprises a linear double-stranded region, wherein the linear double-stranded region comprises a linear portion of the first amplified DNA product and a linear portion of the second amplified DNA product, and wherein the linear double-stranded region is closed at a first end by the first adaptor molecule and closed at a second end by the second adaptor molecule.

7. The method of claim 1, wherein the linear DNA product is an open linear DNA product, wherein the method comprises:
a) amplifying a first DNA template molecule to generate a first amplified DNA product, wherein the first DNA template molecule comprises at least one cleavable target sequence and wherein the first amplified DNA product is a first concatemer, and amplifying a second DNA template molecule to generate a second amplified DNA product, wherein the second DNA template molecule comprises at least one cleavable target sequence and wherein the second amplified DNA product is a second concatemer;
b) forming a single contiguous aqueous volume comprising the first amplified DNA product, the second amplified DNA product, an endonuclease, a ligase, and first and second adaptor molecules; and
c) incubating the single contiguous aqueous volume to generate the open linear DNA product, wherein the linear DNA product comprises a linear double-stranded region, wherein the linear double-stranded region comprises a linear portion of the first amplified DNA product and a linear portion of the second amplified DNA product, and wherein the first adaptor molecule is ligated to a first end of the linear double-stranded region and the second adaptor molecule is ligated to a second end of the linear double-stranded region, and wherein the first and second adaptor molecules are nucleic acid molecules that each comprise one or more nuclease-resistant nucleotides.

8. The method of claim 1, wherein the linear DNA product is a partially closed linear DNA product, wherein the method comprises:
a) amplifying a first DNA template molecule to generate a first amplified DNA product, wherein the first DNA template molecule comprises at least one cleavable target sequence and wherein the first amplified DNA product is a first concatemer, and amplifying a second DNA template molecule to generate a second amplified DNA product, wherein the second DNA template molecule comprises at least one cleavable target sequence and wherein the second amplified DNA product is a second concatemer;
b) forming a single contiguous aqueous volume comprising the first amplified DNA product, the second amplified DNA product, an endonuclease, a ligase, and first and second adaptor molecules; and
c) incubating the single contiguous aqueous volume to generate the partially closed linear DNA product, wherein the partially closed linear DNA product comprises a linear double-stranded region, wherein the linear double-stranded region comprises a linear portion of the first amplified DNA product and a linear portion of the second amplified DNA product, and wherein the first adaptor molecule is ligated to a first end of the linear double-stranded region and the second adaptor molecule is ligated to a second end of the linear double-stranded region, and wherein the first adaptor molecule is a nucleic acid molecule that comprises one or more nuclease-resistant nucleotides, and wherein the linear double-stranded region is closed at the second end by the second adaptor molecule.

9. The method of any one of claims 1-8, wherein the linear double-stranded region comprises a linear portion of the first amplified DNA product ligated to a linear portion of the second amplified DNA product.

10. The method of any one of claims 1-9, wherein the first DNA template molecule is amplified by rolling circle amplification to generate the first amplified DNA product and/or wherein the second DNA template molecule is amplified by rolling circle amplification to generate the second amplified DNA product.

11. The method of any one of claims 1-10, wherein the first amplified DNA product and the second amplified DNA product generated in step (a) are not purified prior to step (b).

12. The method of any one of claims 1-11, wherein the DNA template molecules are amplified either (i) separately, or (ii) together in a single contiguous aqueous volume.

13. The method of any one of claims 1-12, wherein the at least one cleavable target sequence is a restriction endonuclease target sequence and the endonuclease is a restriction endonuclease, optionally where the at least one cleavable target sequence is a Type IIS restriction endonuclease target sequence and the endonuclease is a Type IIS restriction endonuclease.

14. The method of any one of claims 1-12, wherein the endonuclease is an RNA-guided DNA endonuclease.

15. A method for in vitro transcription of a linear DNA product, the method comprising:
(a) providing a linear DNA product, wherein the linear DNA product is produced by the method of any one of claims 1-14;
(b) contacting the linear DNA product with an RNA polymerase; and
(c) producing a transcription product.
